# EUROPEAN PATENT SPECIFICATION

(11) **EP 4 061 424 B1**
(45) Date of publication and mention of the grant of the patent: **03.06.2026**
(21) Application number: 19952755.7
(22) Date of filing: 14.11.2019
(51) Int. Cl.: A61K 47/00, A61K 47/64, A61P 35/00, C07K 14/47, A61K 38/17

(54) **ASC SPECKS IN CANCER IMMUNOTHERAPY**
ASC-FLECKEN IN DER KREBS-IMMUNTHERAPIE
TACHES ASC UTILISÉES EN IMMUNOTHÉRAPIE ANTICANCÉREUSE

(43) Date of publication of application: 28.09.2022
(73) Proprietor: Bogazici Universitesi, 34342 Istanbul (TR)
(72) Inventor: OZOREN, Nesrin, 34337 Istanbul (TR)
(74) Representative: Yalvaç, Oya
(86) International application number: PCT/TR2019/050952
(87) International publication number: WO 2021/096446

(56) References cited:
- WO-A2-01/29235
- US-A1- 2015 141 627
- SAHILLIOGLU ALI CAN ET AL: "Artificial Loading of ASC Specks with Cytosolic Antigens", PLOS ONE, vol. 10, no. 8, 10 August 2015 (2015-08-10), US, pages e0134912, XP093173215, ISSN: 1932-6203, DOI: 10.1371/journal.pone.0134912
- AHMED SHERIF G ET AL: "Gene therapy with apoptosis-associated speck-like protein, a newly described schwannoma tumor suppressor, inhibits schwannoma growth in vivo", vol. 21, no. 7, 11 July 2019 (2019-07-11), US, pages 854 - 866, XP093008858, ISSN: 1522-8517, Retrieved from the Internet <URL:https://watermark.silverchair.com/noz065.pdf?token=AQECAHi208BE49Ooan9kkhW_Ercy7Dm3ZL_9Cf3qfKAc485ysgAAAu4wggLqBgkqhkiG9w0BBwagggLbMIIC1wIBADCCAtAGCSqGSIb3DQEHATAeBglghkgBZQMEAS4wEQQM2_tZdOJMovAyn3bNAgEQgIICoUoA7SSE0IhMQjZTCCp0--KQ_p4Ese8BSyXDcJP8kt009YeB0y7MOFv_SAuR_u-Mi1hmOKYz-Kiq4I2Ahf-6hJ29nqKDn> DOI: 10.1093/neuonc/noz065
- LIU QIANLING ET AL: "Epigenetic inactivation of the candidate tumor suppressor gene ASC/TMS1 in human renal cell carcinoma and its role as a potential therapeutic target", ONCOTARGET, vol. 6, no. 26, 9 September 2015 (2015-09-09), United States, pages 22706 - 22723, XP093008859, ISSN: 1949-2553, DOI: 10.18632/oncotarget.4256
- ZAROUR H. M., DE LEO A., FINN OLIVERA J., , STORKUS WALTER J: "Application of Tumor Antigens for Clinical Immunotherapy", HOLLAND- FREI CANCER MEDICINE, 2003, Hamilton (ON, XP055823145, Retrieved from the Internet <URL:https://www.ncbi.nlm.nih.gov/books/NBK13676>
- FINN, OLIVERA J: "Human tumor antigens yesterday, today, and tomorrow", CANCER IMMUNOLOGY RESEARCH, vol. 5, no. 5, May 2017 (2017-05-01), pages 347 - 354, XP055823147

## Description

### TECHNICAL FIELD

The present invention relates to the use of ASC specks formed by ASC proteins in the treatment of cancer and the compositions of ASC speck formed by ASC proteins and tumor antigens.

### BACKGROUND ART

According to the World Health Organization (WHO), cancer is a leading cause of death worldwide; accounting for an estimated 9.6 million deaths in 2018. The number of new cases has risen to 18 million in the same year, with lung cancer being the most frequently diagnosed cancer type, followed by breast cancer.

Today, cancer treatment options include surgery, radiation therapy, chemotherapy, targeted drug therapy, bone marrow transplant, hormone therapy and immunotherapy. As it is well-known in the art most of these treatment methods have serious side effects and still cannot efficiently cure all the cancer types as it is apparent from the mortality rates. Immunotherapy is relatively safe since it uses the body's own defense mechanism to fight cancer.

Traditional cancer treatment methods are based on the principle of attacking the cancer by chemicals (chemotherapy), radiation or by surgical removal of the tumors. Chemotherapy is a very common method used either as a primary or secondary treatment. It attacks the fast growing cells which include cancer cells and also normal cells such as bone marrow cells. Therefore, as a major side effect, chemotherapy suppresses the immune system of the patient. On the other hand, immunotherapy helps the body's own immune system attack the cancer cells and makes it possible to treat multiple cancer types effectively. Unlike chemotherapy, immunotherapy improves the overall immune system functions.

Immune system is a network of organs, tissues, cells and proteins. The main function of the immune system is to protect the body from invaders such as bacteria, viruses, fungi or any other foreign body. Owing to the research on cancer mechanism it has been known for many years that the cancer cells are also recognized by the immune system. However, since they are derived from the body's own cells, it is not easy for the defense mechanism to identify cancer cells as foreign bodies.

Another important aspect is that for the immune system to successfully protect the body from invaders or abnormal cells, it needs sufficient time to recognize such bodies, produce necessary defense mechanism and destruct them. Considering that the cancer cells are fast growing and also very complex, the defense cells of the body usually shut down before completing their functions on cancer cells. Therefore, it has been accepted that the immune system requires help to be able to protect the body from fast growing cancer cells.

Immunotherapy is a relatively new treatment method with many unknowns. First of all, the role of the immune system in cancer is not completely elucidated. Secondly, the proteins which are involved in this natural defense mechanism are highly sensitive to environmental changes and lose their functions easily. This is a challenge both in the development of new treatment options and also in the use of these treatments on patients.

Recent studies in immunology and tumor biology provide new insights into the understanding of the interaction between the immune system and tumor cells. Many studies have established the importance of T cells to mediate anti-tumor immunity. The process of carcinogenesis leads to changes in the expression of antigens for tumor cells, which can be recognized by host T cells (Dermime, S., A. Armstrong, R. E. Hawkins and P. L. Stern, "Cancer vaccines and immunotherapy", British medical bulletin, Vol. 62, No. 1, pp. 149-162, 2002).

Currently, immune checkpoint inhibitors, engineered T cells, monoclonal antibodies and cancer vaccines are being commonly used as immunotherapy against many cancer types (Kamta, J., M. Chaar, A. Ande, D. A. Altomare and S. Ait-Oudhia, "Advancing cancer therapy with present and emerging immuno-oncology approaches", Frontiers in oncology, Vol. 7, p. 64, 2017).

Cancer vaccines can be divided into two main groups; prophylactic or therapeutic vaccines. Theoretically, prophylactic cancer vaccines should prevent development of tumors and offer a long-lasting immunity. The first examples of prophylactic vaccines are against the infection of HBV and HPV (Chang, M.-H., "Hepatitis B Virus and Cancer Prevention", Recent results in cancer research. Fortschritte der Krebsforschung. Progres dans les recherches sur le cancer, Vol. 188, pp. 75-84, 2010).

Therapeutic vaccines can be divided into 4 main subgroups according to their composition; whole-cell tumor, dendritic cell, DNA and subunit vaccines. Whole- cell tumor vaccines are mostly allogenic and have a limited success because of the large scale of observed tumoral heterogeneity between patients (Srivatsan, S., J. M. Patel, E. N. Bozeman, I. E. Imasuen, S. He, D. Daniels and P. Selvaraj, "Allogeneic tumor cell vaccines", Human Vaccines & Immunotherapeutics, Vol. 10, No. 1, pp. 52-63, 1 2014). Dendritic cell vaccines are produced by extraction of the cells from the patient and stimulation of them with specific antigens and/or adjuvants (Kamta, J., M. Chaar, A. Ande, D. A. Altomare and S. Ait-Oudhia, "Advancing Cancer Therapy with Present and Emerging Immuno-Oncology Approaches.", Frontiers in oncology, Vol. 7, p. 64, 2017). There is one FDA approved dendritic cell vaccine, Sipuleucel, against prostate cancer (Guo, C., M. H. Manjili, J. R. Subjeck, D. Sarkar, P. B. Fisher and X.-Y. Wang, "Therapeutic Cancer Vaccines", Advances in cancer research, Vol. 119, pp. 421-475, 2013). DNA vaccines are newly emerging in cancer immunotherapy and they basically need a sequence of a tumor associated antigen.

Subunit vaccines for cancer immunotherapies include antigens or epitopes of antigens as in classic vaccines. There are applications of subunit vaccines to cure melanoma, prostate, testicular and breast cancer. Although the immune booster effect of subunit vaccines are milder than whole-tumor vaccines, it is possible to increase their effect with adjuvants (Temizoz, B., E. Kuroda and K. J. Ishii, "Vaccine adjuvants as potential cancer immunotherapeutics", International immunology, Vol. 28, No. 7, pp. 329-38, 2016).

Vaccines aim to induce adaptive immunity which can be enhanced by using adjuvants in certain vaccine formulations. Mineral salts, cytokines, emulsions, microbial particles and liposomes can be used as adjuvants (Guy, B., "The perfect mix: recent progress in adjuvant research.", Nature Reviews Microbiology, Vol. 5, No. 7, pp. 505-17, 6 2007). Action mechanisms of adjuvants have just started to be enlightened even though they have been used for more than 80 years (Awate, S., L. A. Babiuk and G. Mutwiri, "Mechanisms of action of adjuvants.", Frontiers in immunology, Vol. 4, p. 114, 2013).

Aluminum salts (Alum) are the most frequently used vaccine adjuvants in human vaccines. The induction effect of aluminum hydroxide for diphtheria was firstly shown in 1926 in guinea-pigs and different formulations of Alum have been used in human vaccines since 1931 (Marrack, P., A. S. McKee and M. W. Munks, "Towards an understanding of the adjuvant action of aluminium.", Nature reviews. Immunology, Vol. 9, No. 4, pp. 287-93, 2009).

However, the action mechanism of Alum adjuvants could not be understood completely, to date. A few recent publications show that Alum induces immune system over IL-1 cytokines and caspase 1, which is the product of the inflammasome complex (Franchi, L., T. Eigenbrod, R. Munoz-Planillo and G. Nunez, "The inflammasome: a caspase-1-activation platform that regulates immune responses and disease pathogenesis.", Nature immunology, Vol. 10, No. 3, pp. 241-7, 3 2009; .10, Eisenbarth, S. C., O. R. Colegio, W. O'Connor, F. S. Sutterwala and R. A. Flavell, "Crucial role for the Nalp3 inflammasome in the immunostimulatory properties of aluminium adjuvants", Nature, Vol. 453, No. 7198, pp. 1122-1126, 6 2008; Grun, J. L. and P. H. Maurer, Different T helper cell subsets elicited in mice utilizing two different adjuvant vehicles: the role of endogenous interleukin 1 in proliferative responses.",Cellular immunology, Vol. 121, No. 1, pp. 134-45, 6 1989).

More than 70 years after Alum, AS04, a new adjuvant, was licensed by FDA for HPV. AS04 is formed from a combination of Alum and monophosphoryl lipid A. Lastly, FDA approved the usage of AS03 for pandemic H5N1 influenza, but it is not commercial anymore. The formulation of MF59 which is used for influenza vaccines is very similar to AS03 but it is not licensed by FDA (Awate, S., L. A. Babiuk and G. Mutwiri, "Mechanisms of action of adjuvants.", Frontiers in immunology, Vol. 4, p. 114, 2013).

There are a number of proposed mechanisms about the booster effect of adjuvants in immune activation which are basically enhancement of antigen uptake, immune cell recruitment to site of injection, activation of PRRs and inflammasome followed up by particular immune response such as Th1 and Th2 (Reed, S. G., M. T. Orr and C. B. Fox, "Key roles of adjuvants in modern vaccines", Nature Medicine, Vol. 19, No. 12, pp. 1597-1608, 12 2013).

Receptors of the innate immune system like TLRs are the targets of adjuvants. When an adjuvant activates TLRs, certain chemokines and cytokines are secreted to the extracellular matrix. Then, these small molecules help the orientation of cellular and humoral immunity. For example, secretion of IL-4 from APCs and naive CD4 cells directs immunity to induce production of IgG, as IL-12 and IFN- cause more cellular immune response (Reed, S. G., M. T. Orr and C. B. Fox, "Key roles of adjuvants in modern vaccines", Nature Medicine, Vol. 19, No. 12, pp. 1597-1608, 12 2013).

Innovative approaches have been developed after the identification of a certain signature antigen on human tumor cells resulting in the development of antigen specific vaccination. Combination of certain tumor antigens and proper routes for the delivery of these antigens to the immune system aims for optimizing human-based vaccination (Dermime, S., A. Armstrong, R. E. Hawkins and P. L. Stern, "Cancer vaccines and immunotherapy", British medical bulletin, Vol. 62, No. 1, pp. 149-162, 2002).

Tumor cells can also be used as vaccines. Since these cells as such are not able to cause adequate immune response, researchers focus on overcoming this unresponsiveness by introducing co-stimulatory molecules or cytokine genes into the genome of tumor cells which are then used as vaccines and given back to the same patient. However, this kind of vaccine production is rather slow, labor-intensive and difficult in a logistic manner.

Alternatively, recombinant viral vectors can be used for the delivery of tumor antigen to the body. The intrinsic ability of viral vectors for the initiation of immune response and the formation of inflammatory reactions as a result of viral infection are the advantages of these vaccines. However, a viral vector ideally should be safe and should not promote anti-vector immune responses to allow the boosting of anti-tumor specific immune responses (Dermime, S., A. Armstrong, R. E. Hawkins and P. L. Stern, "Cancer vaccines and immunotherapy", British medical bulletin, Vol. 62, No. 1, pp. 149-162, 2002).

Despite the cancer treatment methods known in the art, incidence of the cancer and in parallel to this, the mortality rates are increasing each year. Therefore, there is still a huge need in the art for a new cancer treatment method.

ASC (Apoptosis Associated speck-Like Protein Containing CARD) is a 22 kDa adaptor protein with a PYD domain at N-terminal and CARD domain at C-terminal that functions in the formation of inflammasome complexes, particularly in NLRP3, NLRC4 and AIM2 inflammasomes. ASC protein has a role in the activation of caspase-1 protein in inflammatory and pyroptotic signaling pathways (Franchi et al., 2009). It is a cytoplasmic protein that forms a speck like structure upon the activation of inflammasome (Miao et al., 2011).

ASC proteins polymerize into filaments and form a large supramolecular scaffold formation called as ASC speck (Sahillioglu, A. C., F. Sumbul, N. Ozoren and T. Haliloglu, "Structural and dynamics aspects of ASC speck assembly", Structure, Vol. 22, No. 12, pp. 1722-1734, 2014). These ASC specks can exist inside the cell or can be released to the outside (Sahillioglu, A. C. and N. Ozoren, "Artificial loading of ASC specks with cytosolic antigens", PloS one, Vol. 10, No. 8, p. e0134912, 2015).

The NLRP3 inflammasome, which also contains the ASC protein, can be triggered by stimuli such as extracellular ATP, membrane damaging toxins (nigericin), lysosomal damage, monosodium urea (MSU) and UV rays. NLRP3 inflammasome activation is accompanied by the synthesis of ASC specks. It has been shown that ASC specks can be synthesized in vitro by incubating purified recombinant ASC protein in a hypotonic solution (Fernandes-Alnemri et al., 2007).

The international patent application WO 2009/014863 disclosed that the ASC protein and the pyroptosome could be used in the treatment and diagnosis of autoimmune diseases. According to this application, apoptosome and pyroptosome structures containing ASC dimers and procaspase-1 occur during the early inflammatory response in macrophage cells. Caspase-1 activation is dependent on pyroptosome formation. The state of inflammation is diagnosed by isolating pyroptosomes from macrophage cells.

After MF59-adjuvanted influenza vaccination, it was postulated that the ASC protein plays a role in triggering antigen specific humoral response (Ellebedy et al., 2011).

In another prior art disclosure, Sherif, A. G. et al, Neuro-Oncology 2019, 21(7), 854-866, an apoptosis-associated speck-like protein containing a caspase recruitment domain (ASC) by itself was explored as a schwannoma tumor suppressor in a schwannoma gene therapy regimen that can be translated to clinical use. In this document, the researchers have made an intratumoral injection of adeno-associated virus vector encoding ASC in a human xenograft schwannoma model. In this experiment, reduction in tumor growth was seen upon injection and further tumor-associated pain was resolved without significant toxicity.

One other prior art document related to the invention is, Liu, Q. et al, Oncotarget 2015, 6(26), 22706-22723, where a study investigating the biological function of the pro-apoptotic gene ASC/TMS1 in renal cell carcinoma is published. This work shows that a significant downregulation og ASC/TMS1 was detected in renal tumors compared to adjacent non-cancerous tissues. It was also shown that re-expression of ASC/TMS1 in silenced renal cell carcinoma cell lines inhibited cell viability and arrested cell cycle while inducing apoptosis and repressing tumorigenicity showing an antitumorigenic function of ASC/TMS1 alone in renal cancer.

Additionally, WO 01/29235 is disclosing TMS1 compositions which is a synonym for ASC speck. In this document, it was disclosed that TMS1 was abnormally silenced in a large portion of the human breast cancer cell samples which implies that TMS1 has a tumor suppressor function. It was further seen thatTMS 1 was silent in some other human tumor cells such as Molt 4 lymphotic leukemia cells or HeLa cervican carcinoma cells. The researchers this work further disclosed a method of treating cancer where TMS1 is administered together with an anti-cancer therapy.

Furthermore, with the invention disclosed in the patent application WO 2013/160807, it has been found that the ASC speck carriers can be loaded with antigens and/or bioactive molecules so as to be used for vaccination. Said invention has been developed by the inventor of the present invention. There, it has been found that the stability of bioactive molecules can be enhanced by ASC specks as carriers for at least 30 days and they can endure at least 8 freeze-thaw cycles without degradation. In other words, the mentioned application discloses a composition useful for vaccine in general. However, the therapeutic effect of the ASC specks as such or the composition comprising an ASC speck for the treatment of cancer cells or tumors has not been disclosed.

Consequently none of the prior art documents mention the role of ASC specks in the treatment of cancer.

### SUMMARY OF THE INVENTION

The aim of the present invention is to provide a new cancer treatment method.

The present invention is directed to the use of a tumor antigen and an ASC speck formed by Apoptosis Associated Speck-Like Protein Containing CARD (ASC proteins) in the cancer immunotherapy.

In one embodiment of the present invention a composition comprising an ASC speck formed by ASC proteins and a tumor antigen is provided for use in the therapeutic treatment of cancer cells or tumors.

In another embodiment the present invention provides composition for generating an immune response to cancer in a mammal, comprising a tumor antigen and an ASC speck formed by ASC proteins.

In another embodiment of the present invention a tumor antigen and an ASC speck formed by ASC proteins are used in the treatment of cancer with their ability to boost the immune system against tumor cells.

Furthermore, in another embodiment of the present invention a composition comprising a combination of ASC speck formed by ASC proteins and a tumor antigen is provided for use in the treatment of cancer wherein said method comprises inducing and/or stimulating an immune response against a cancer disease in a mammal, particularly in a human subject, wherein said induction or stimulation causes a decrease in cancer progression or growth arrest of cancer cells.

### Brief Description of the Figures

**Figure 1****.** Production and purification of tOVA-ASC specks from HEK293FT cell line. a) tOVA-ASC encoding p-C3-d (1-238) OVA-ASC plasmid. Coomasie blue staining (b), Anti-hASC blot (c) and bright field image (d) of purified tOVA-ASC specks. e) Florescent image of purified mCherry-ASC specks.
**Figure 2****.** Engulfment of mCherry-ASC specks by PMA differentiated THP-1 macrophages. a) Bright field and florescent images of THP-1 macrophages was taken 12 hours after treatment. b) Anti-hASC blot of THP-1 macrophages treated with PBS, mCherry and mCherry-ASC. c) Flow cytometry analysis of engulfed mCherry-ASC specks.
**Figure 3****.** Degradation of engulfed mCherry-ASC specks by THP-1 macrophages. Florescent image of engulfed and degraded mCherry-ASC specks was taken 12 hours after mCherry-ASC treatment.

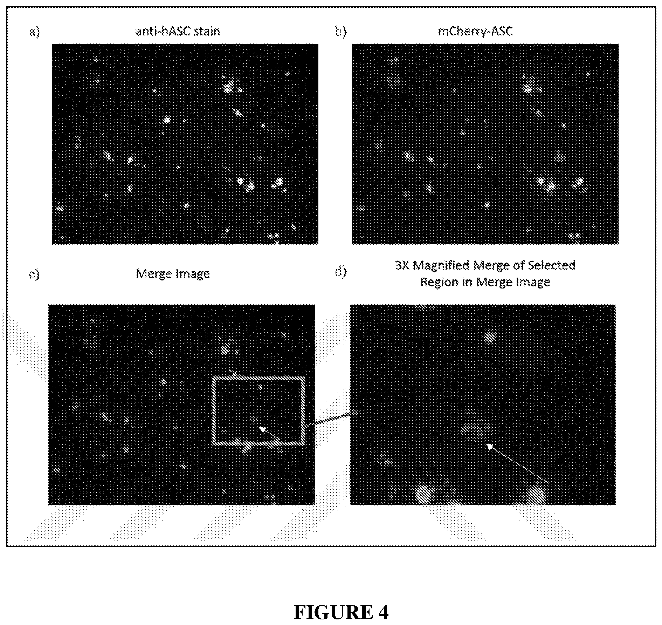

**Figure 4****.** Complete degradation of mCherry-ASC specks resulted in formation of inflammasome complex in host cell. Florescent (a-b) and merge images (c) of anti-hASC staining of engulfed mCherry-ASC specks by THP-1 macrophages. d) 3X magnified image of blue frame in the merge image.
**Figure 5****.** Secretion of pro-inflammatory cytokines by mCherry-ASC treatment. a) mRNA expression levels of NLRP3, pro-caspase-1 and ASC with RT-qPCR, IL-1β (b) and TNF- α (c) secretion levels were determined after mCherry-ASC treatment of THP-1 macrophages. (Error bars show SD value between two biological replica.)
**Figure 6****.** Engulfment of tOVA-ASC specks by PMA differentiated THP-1 Macrophages. a) immunocytochemistry of THP-1 macrophages 12 h after tOVA-ASC, PBS, OVA with anti-hASC antibody. b) Anti-OVA and c) anti-hASC blot of THP-1 macrophages after PBS, OVA and tOVA-ASC treatments.
**Figure 7****.** Secretion of pro-inflammatory cytokine from PMA differentiated THP-1 macrophages upon tOVA-ASC treatment. IL-1 β (a), TNF- α (b) and IL-6 (c) secretion levels of THP-1 macrophages treated with PBS, 10 µg OVA, 2 µg, 5 µg and 10 µg tOVA-ASC specks was determined by ELISA.
**Figure 8****.** Cytokine secretion levels from splenocytes of tOVA-ASC injected animals after OVA pulsing. The splenocytes either pulsed with OVA or non-pulsed. Cytokine levels of the splenocytes was determined from supernatant of the cells by ELISA. (n=4 for OVA group and n=6 for tOVA-ASC group.)
**Figure 9****.** Chicken ovalbumin producing EG7-OVA cell line used for tumor model. a) Anti-OVA blot of EG7-OVA cell line. b) Timeline of EG7-OVA tumor model model experiment. (OVA: 43kDa)
**Figure 10****.** Progression of EG7-OVA tumors after tOVA-ASC injections. a) Average tumor volume for all injections after injections. (Each dot represents one tumor.) b) Progression of each tumor in different groups, separately. c) Average weight of excised tumors. (n=3 for PBS, n=5 for OVA and n=8 for tOVA-ASC injections.)
**Figure 11****:** Eradication of subcutaneously inoculated EG7-OVA tumors after two shot intraperitoneal injection of tOVA-ASC specks. Photographs of excised tumors from PBS, OVA and tOVA-ASC injected animals were taken. 6 out of 8 tumors in tOVA-ASC injected animals had been completely eradicated.
**Figure 12****.** OVA specific IgG Response of 100 µg tOVA-ASC injected C57BL/6 mice carrying EG7-OVA tumors. Anti-OVA IgG response was determined from plasma taken just before sacrificing the animal by ELISA. (n=1 for PBS, n=4 for OVA and n=6 for tOVA-ASC injections.)
**Figure 13****.** Intraperitoneally injected tOVA-ASC speck was detected in subcutaneously inoculated EG7-OVA tumor. Anti-TMS1 blot of tumor samples of tOVA-ASC, OVA and PBS injected animals.
**Figure 14****.** CD11C+ dendritic cells infiltration into EG7-OVA tumors of tOVA-ASC injected animals. 50 um sections of tumors of PBS, OVA and tOVA-ASC injected animals was taken with cryostat and stained with anti-CD11c antibody. (CD11c: Dendritic cell marker. White arrows show CD11c+ cells and red arrows show non-specific antibody remnants in gap of the tumor tissue.)
**Figure 15****.** T cell expansion in spleen of tOVA-ASC injected animals. Splenocytes from excised spleens of PBS and tOVA-ASC injected animals was stained with anti-CD4, anti-CD8 and anti-B220 antibodies and percentage of the cells were analyzed with ow cytometer. (CD4: T helper cell, CD8: Cytotoxic T cell, B220: B cell marker; Error bars show SD value between animals in tOVA-ASC group.)
**Figure 16****.** Intraperiteonally injected mCherry-ASC was located in mesenteric lymph node of one animal. PBS, mCherry and mCherry-ASC injected animals were anesthetized and exited at 580 nm under IVIS machine at day 1 (a) and at day 2 (b).
**Figure 17****.** Drainage of intraperiteonally injected mCherry-ASC specks to secondary lymphoid organs. Lymph node (A) and spleen (B) of PBS, mCherry and mCherry-ASC injected animals were lysed via sonication and blotted with anti-TMS-1 antibody.
**Figure 18****.Vaccination** timeline for immunization of mice that had EG7-OVA tumors.
**Figure 19****.** Volume of tumors after immunizations. After immunizations tOVA-ASC group had the highest anti-tumor activity comparing to other groups. mCh-ASC+OVA group gave the discussible result because 1 of them had completely eradicated tumor after immunizations. Each dots represents each mice (n=4).
**Figure 20****.** OVA specific IgG response for experimental groups of EG7-OVA model. OVA specific IgG response was the highest for tOVA-ASC immunized mice. Comparing to OVA group mCh-ASC+OVA group had higher OVA specific IgG levels. There was significant increase in the level of IgG for comparison of OVA, mCh-ASC+OVA and tOVA-ASC groups to PBS group. (n=4 for each group)

### DETAILED DESCRIPTION OF THE INVENTION

The main object of the present invention is to develop a novel cancer immunotherapy method. This is achieved by the use of ASC protein, which has critical functions in inflammatory signaling pathways.

ASC protein, also referred to as PYCARD, is an adaptor protein encoded by the PYCARD gene. It has an N terminal PYRIN domain, C terminal caspase recruitment domain (CARD) and a linker region between them. ASC proteins form a bridge between pro-caspase-1 via CARD-CARD interactions and nucleotide binding oligomerization domain-like receptor (NLR) proteins via PYRIN-PYRIN interactions.

NLR family of proteins in the cytosol sense pathogen associated molecular patterns (PAMPs) and danger-associated molecular patterns (DAMPs). Certain NLRs like NLRP3 and AIM2 induce the formation of inflammasome complexes. Caspase-1 is activated within the inflammasome multiprotein complex via interaction with ASC proteins. Activated caspase-1 cleaves pro-IL-1beta and pro-IL-18 to their mature forms and these cytokines are released to the extracellular space to induce inflammation.

In unstimulated cells, ASC is soluble in the cytosol. Upon stimulation ASC proteins come together and form a globular structure called as ASC speck. ASC specks can be secreted to the outside of the cell to accelerate the recruitment of immune cells.

For the purposes of the present invention Chicken Ovalbumin Protein (OVA) is used as a model antigen.

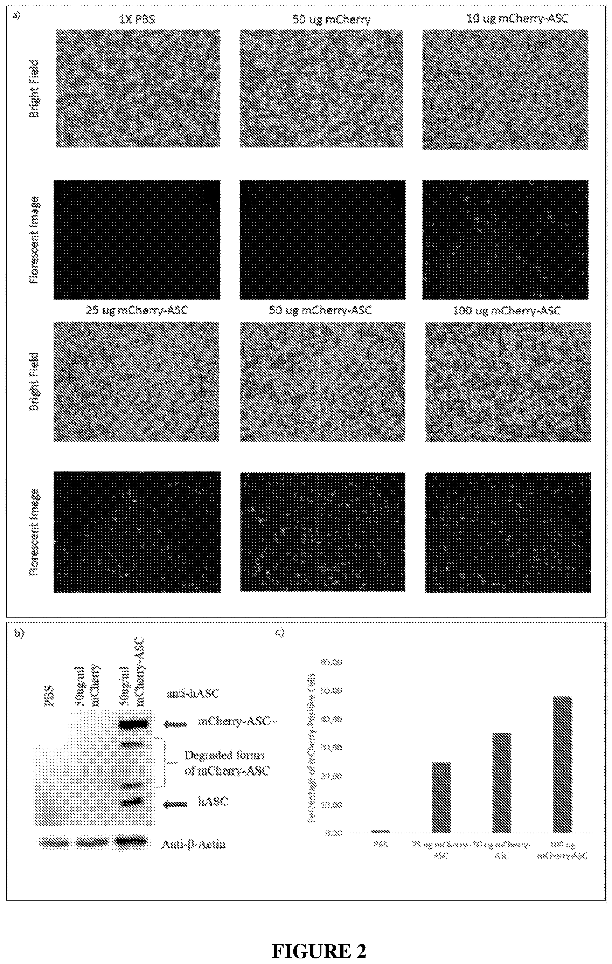

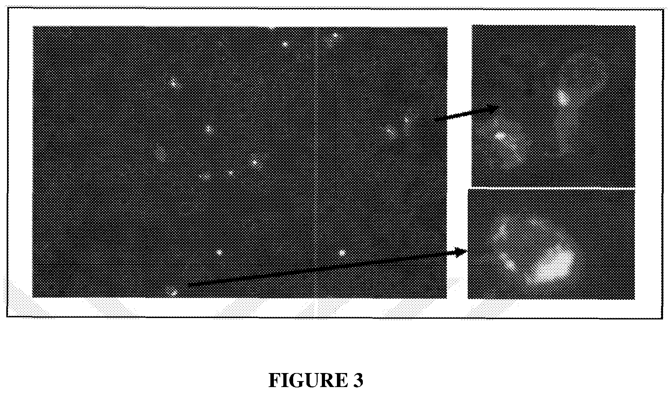

Since one of the molecular action mechanisms of current adjuvants is the enhancement of antigen uptake by APCs, THP-1 monocytic cell line was used for cell culture experiments. In order to understand whether ASC specks had been engulfed by APCs, PMA differentiated THP-1 macrophages were subjected to tOVA-ASC specks and mCherry-ASC specks, separately. With this experiment, it has been shown that both tOVA-ASC specks and mCherry-ASC specks are engulfed by macrophages in a concentration dependent manner (Figure 2). Moreover, it has been shown that engulfed mCherry-ASC specks are degraded by THP-1 macrophages and found at different locations in the cell with florescent and confocal microscope (Figure 3).

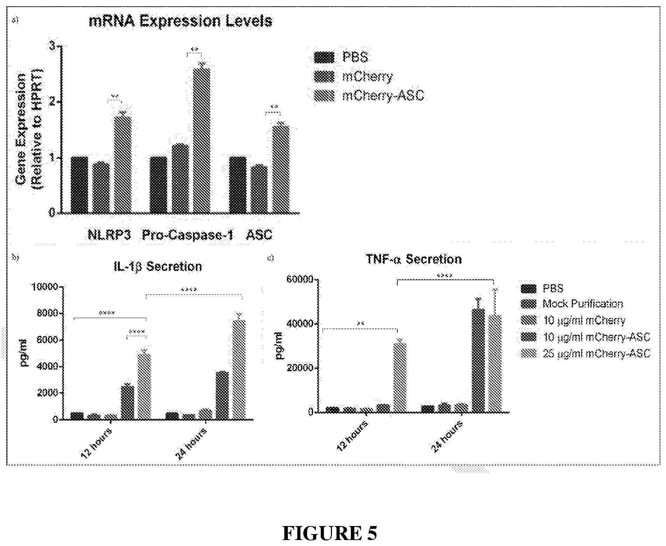

Inflammasome is one of the target of commonly used adjuvant, Alum, and currently produced adjuvants AS03 and MF59. To understand whether the proposed adjuvant, ASC specks, according to the present invention can cause inflammatory response in THP-1 macrophages, change in mRNA expression levels of pro-inflammatory proteins; NLRP3, ASC and pro-caspase-1 were determined with RT-qPCR. In this regard, it is shown that the expression levels of pro-inflammatory proteins increases at least 1.5 fold after mCherry-ASC speck treatment when it is compared with only mCherry protein treatment (Figure 5).

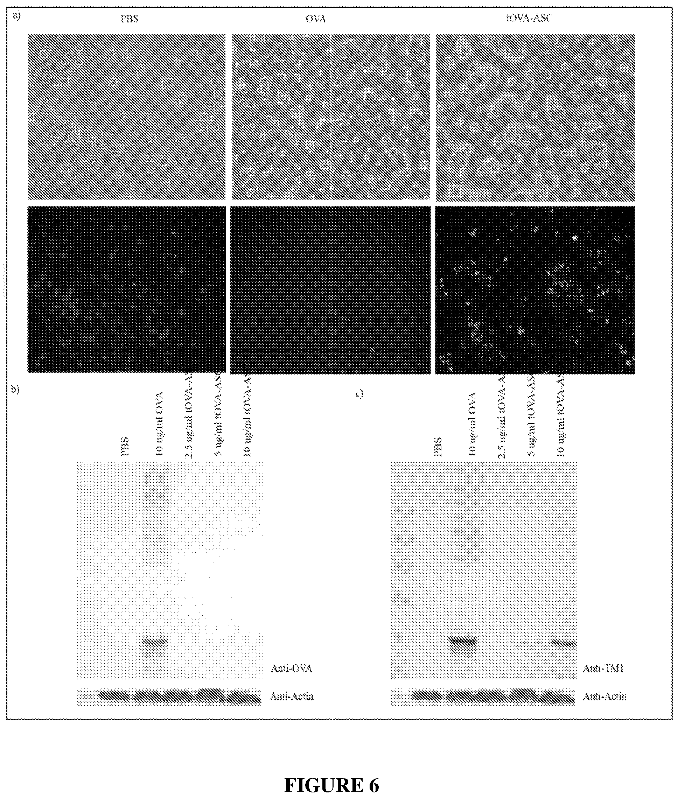

To support RT-qPCR data, IL-1 β, TNF- α and IL-6 cytokine secretion levels were determined by ELISA after treatments with either mCherry-ASC specks or tOVA-ASC specks. IL-1β secretion was increased in a time and concentration dependent manner. Increase in secretion level of TNF-α was in a concentration dependent manner at early times (12 hours after treatment) whereas no difference was observed in secretion level of the TNF-α at late time points (24 hours after treatment) (Figure 6). tOVA-ASC treatments augmented IL-1β secretion in a concentration dependent manner. tOVA-ASC speck treatment at each concentration cause more IL-1β than 10 ug OVA treatment. TNF-α and IL-6 levels are increased in a concentration dependent manner after tOVA-ASC treatment and secretion level of the cytokines are higher than 10 ug OVA treatment. Consequently, ASC specks formed by fusion of antigens with ASC cause inflammatory cytokine secretion from THP-1 macrophages (Figure 6). With these RT-qPCR and ELISA data, it is concluded that ASC speck treatment causes increase in inflammatory response in macrophages.

Antibody titers is not the only criteria for a good vaccine and cytokine production by CD4+ cells can be more powerful correlate of good immunization (Plotkin, 2010). In order to understand cytokine production of T helper cells caused by tOVA-ASC specks in immune cells, ex vivo recall assay was performed and TNF- α, IFN-gamma and IL-4 levels were measured with and without OVA pulsing. IFN-gamma is a cytokine produced mainly by T lymphocytes and natural killer cells and plays an important role in inhibition of tumor progression and metastasis (Farrar, M. A. and R. D. Schreiber, "The Molecular Cell Biology of Interferongamma and its Receptor", Annual Review of Immunology, Vol. 11, No. 1, pp. 571-611, 4 1993). It has been also used as a direct anti-viral agent. It is produced specifically by type 1 T helper cells after vaccination and causes a delayed-type hypersensitivity reactions and as a consequence cell mediated immunity (Romagnani, S., "Type 1 T helper and type 2 T helper cells: functions, regulation and role in protection and disease.", International journal of clinical & laboratory research, Vol. 21, No. 2, pp. 152-8, 1991 ; Godfroid, J., G. Czaplicki, P. Kerkhofs, V. Weynants, G. Wellemans, E. Thiry and J. J. Letesson, "Assessment of the cell-mediated immunity in cattle infection after bovine herpesvirus 4 infection, using an in vitro antigen-specific interferon-gamma assay.", Veterinary microbiology, Vol. 53, No. 1-2, pp. 133-41, 11 1996.) With ex vivo recall assay, the inventors of the present invention have shown that both OVA pulsed and non-pulsed splenocytes of tOVA-ASC speck injected animals secreted IFN-gamma and secretion level decreases after OVA pulsing whereas the other groups secrete IFN-gamma only after OVA pulsing. In this regard, it is considered that tOVA-ASC specks can cause a formation of cellular immune response without antigen specificity.

TNF- α is a pro-inflammatory cytokine and can be required for formation of both humoral and cellular immune response. However, it also cause loss of function in NK cells and thereby decreased secretion of IFN-gamma and cytotoxic activity against certain types of cancer (Romero-Reyes, M., C. Head, N. A. Cacalano and A. Jewett, "Potent induction of TNF-α during interaction of immune effectors with oral tumors as a potential mechanism for the loss of NK cell viability and function", Apoptosis, Vol. 12, No. 11, pp. 2063-2075, 9 2007.)

In the experiment of the present invention, splenocytes of each animal secrete TNF- α after OVA pulsing but the secretion from both splenocytes of tOVA-ASC and Alum+OVA injected animals was less than that of OVA injected animals.

IL-4 cytokine is mainly produced by type 2 T helper cells and known as a mutual antagonist of IFN-gamma. It regulates B cell growth and eosinophil recruitment in vivo (Smiley, S. T. and M. J. Grusby, "Interleukin 4", Encyclopedia of Immunology, pp.1451-1453, Elsevier, 1998.) Secretion of IL-4 from splenocytes of each animal decreases after OVA pulsing however splenocytes of tOVA-ASC injected animals secrete the highest amount of the cytokine with and without OVA pulsing.

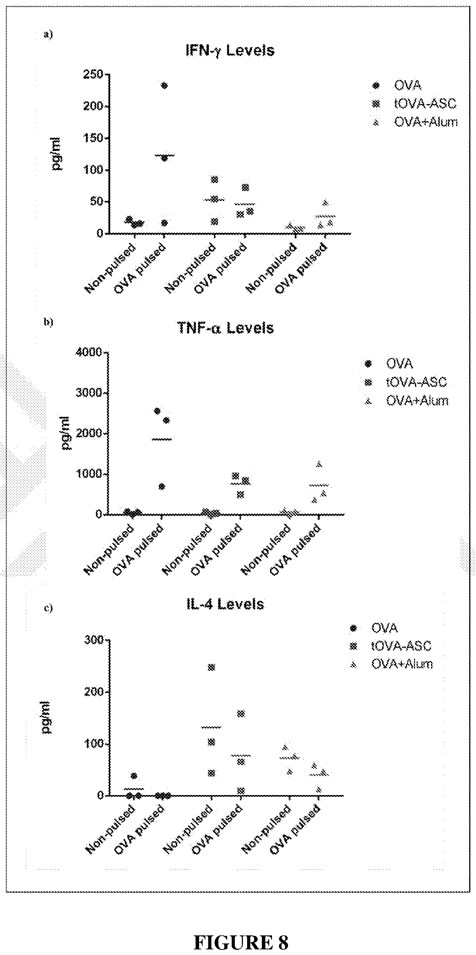

Consequently, the inventors of the present invention showed that TNF- α and IL-4 secretion level from splenocytes of tOVA-ASC injected animals is very similar to the ones injected with commonly used adjuvant Alum+OVA (Figure 8). The difference between proposed adjuvant, ASC specks, and Alum adjuvant is the secretion of IFN-gamma before OVA pulsing. Since IFN-gamma cause more cell mediated immune response, ASC specks can be used to boost cellular immunity.

As mentioned above, immunotherapy is one of the mostly studied research area against cancer. Vaccine adjuvants have been tried to develop to boost cellular immune response of the patient against cancer and IFN-gamma is one of the major cytokine that direct immune system mostly towards cellular immunity part. Since tOVA-ASC specks cause IFN-gamma secretion from splenocytes, the in vivo effect of tOVA-ASC specks on tumor formation was checked by conducting EG7-OVA tumor model in C57BL/6. For this purpose, 2.5 106 EG7-OVA cell was subcutaneously inoculated to the dorsal flank of the mice and the intraperitoneal tOVA-ASC speck injections was started when tumors reached a palpable size. Since tumor sizes showed a heterogeneity and number of the animals was limited, mice were divided into experimental and control groups as each group at least have one small, one middle size and one large tumor. PBS and OVA injections were done as negative control.

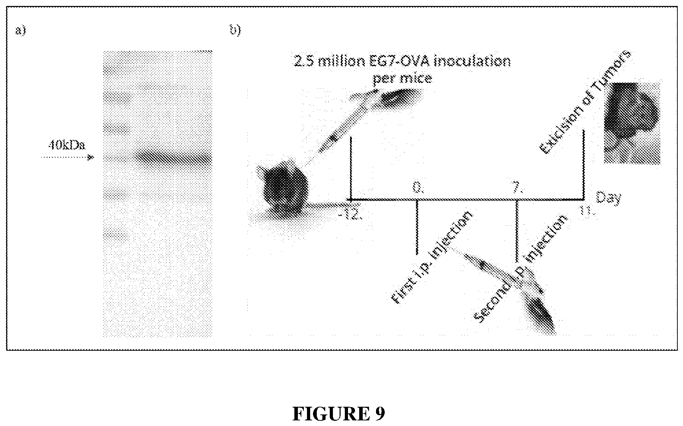

Four tumors in tOVA-ASC injected animals were completely eradicated seven days after first injection, whereas one more tumor was formed in OVA injected animal during the same time. Then, booster injection was done at day seven. Two days after booster injection two more tumors were completely eradicated in tOVA-ASC group. One animal died in PBS group. Since progression of tumors on tOVA-ASC injected animals showed that tOVA-ASC specks somehow have therapeutic effects on EG7-OVA tumors and there was only 2 tumors remained in tOVA-ASC group, the experiment was ended to understand cellular and molecular mechanism of this therapeutic effect of ASC specks. During the necropsy of the animals, only connective tissue but not any remnant tumor tissue was encountered in where tumor inoculation for the animals which tumors had eradicated completely before necropsy. Average weight of the tumors in PBS and OVA group was about 2 g whereas it was 0.03 g in tOVA-ASC group (Figure 9).

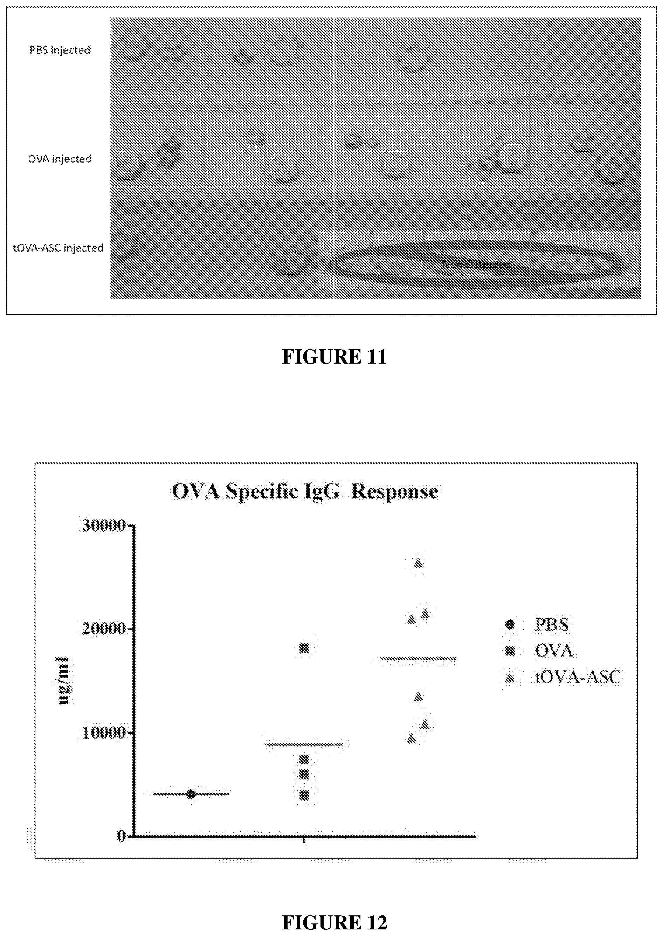

Further experiments with sera of the animals showed that OVA specific IgG response was successfully increased to approximately 18 ug/ml in tOVA-ASC injected animals as it was 5 ug/ml and 8 ug/ml for PBS and OVA groups, respectively (Figure 12). The increase in IgG response of the tOVA-ASC group might be caused by mainly two reasons. Firstly, since EG7-OVA tumors produce continuously OVA protein, if tOVA-ASC specks cause a general immune burst in the mice, it might cause antibody production against OVA. Secondly, it might be caused by doubled amount of intraperitoneally injected tOVA-ASC specks.

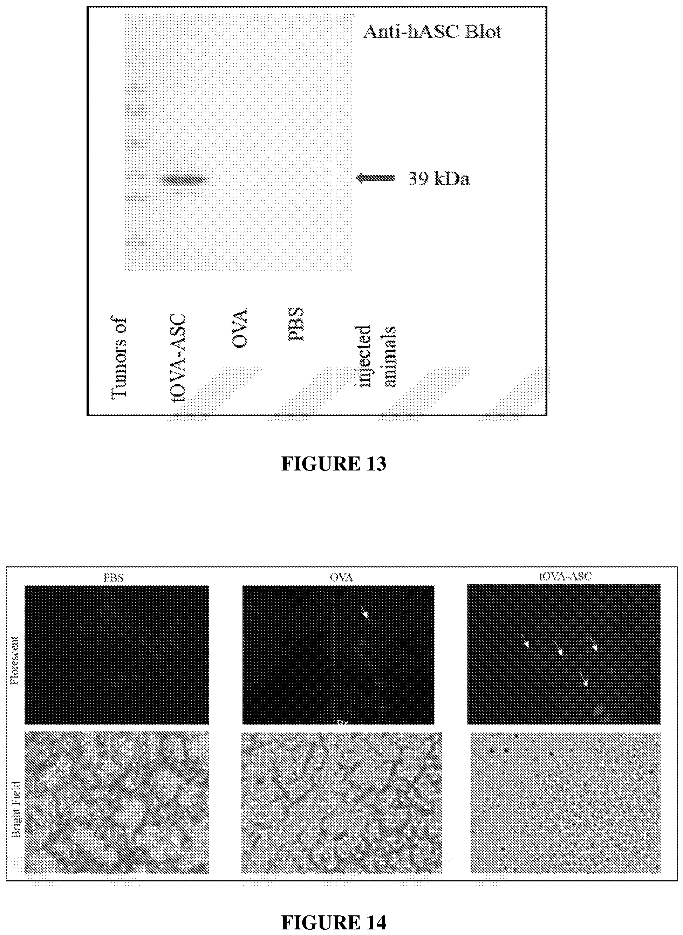

Moreover, the inventors of the present invention recognized intraperitoneally injected tOVA-ASC specks migrated to subcutaneously inoculated EG7-OVA tumor site in one of the animals and remained there as intact at least in tOVA-ASC protein form (Figure 13). Basically, it might be caused by tOVA-ASC specks enter circulation from intraperitoneal site and leak to the tumors because of intensive vascularization around tumor microenvironment and leakage of these blood vessel. The other reason might be that tOVA-ASC specks were engulfed by APCs and these APCs somehow accumulate in tumors. Since tumor infiltrating mature DCs confer an increase in immune activation and recruitment of immune effector cells, it has been checked whether DCs infiltrate into the tumors with IHC staining. CD11C+ antibody was used which stains conventional/classical DC subtype.

This subtype has a defined characteristic morphology with MHC class II expression (Collin, M., N. McGovern and M. Haniffa, "Human dendritic cell subsets.", Immunology, Vol. 140, No. 1, pp. 22-30, 9 2013.) Inventors of the present invention have shown that CD11C+ DCs were localized in tumor cryostats of one of the animals injected with tOVA-ASC [Figure 14]. Interestingly, tOVA-ASC was found in the CD11C+ DC infilltrating tumor. IHC and Western blot data increase the probability that tOVA-ASC specks were carried to tumor microenvironment by DCs.

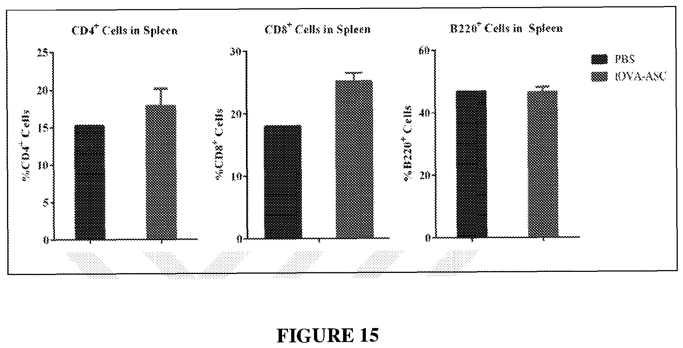

EG7-OVA tumor model was repeated to understand molecular and cellular mechanism of therapeutic effect of tOVA-ASC specks. EG7-OVA cells were inoculated to each dorsal flank of 24 mice but only 4 tumors were formed. To understand effect of tOVA-ASC specks on immune cells expansion and tumor infiltration, PBS and tOVA-ASC were injected to the animals carrying these 4 tumor. Seven days after first injection, one tumor of tOVA-ASC injected animals started to shrink and at day 10 it was not palpable, anymore. Then, experiment was ended to determine percentage of helper T cell, cytotoxic T cell and B cells in spleen with ow cytometry analysis. Proportion of CD4+ and CD8+ cells in spleen of tOVA-ASC injected animals was increased when compared that of PBS injected animal (Figure 15). Both with cytokine secretion from splenocytes and ow cytometry analysis, it can be concluded that tOVA-ASC injections cause a formation of helper T cell activation in C57BL/6 mice.

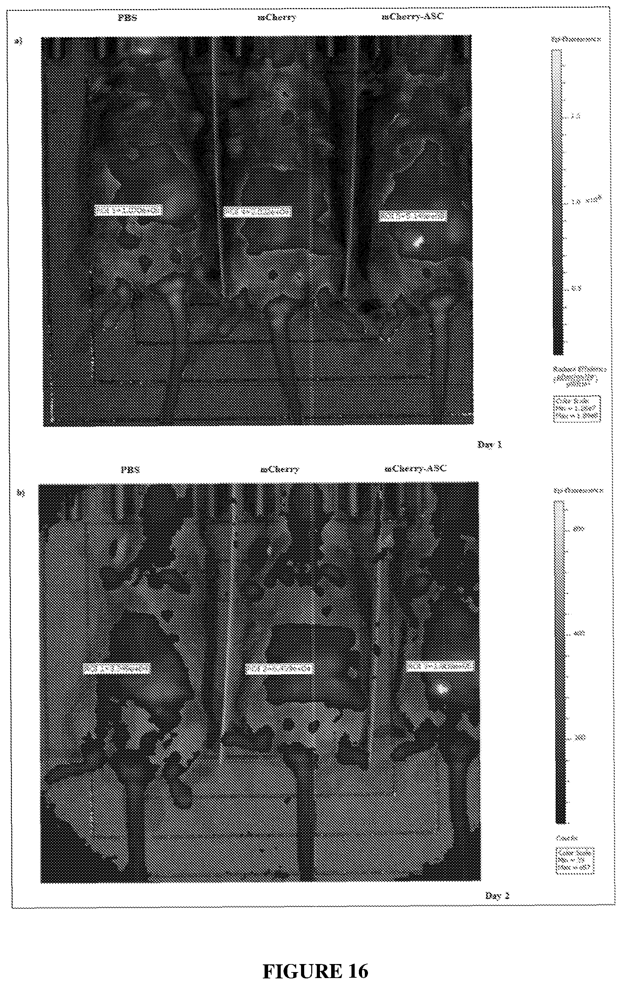

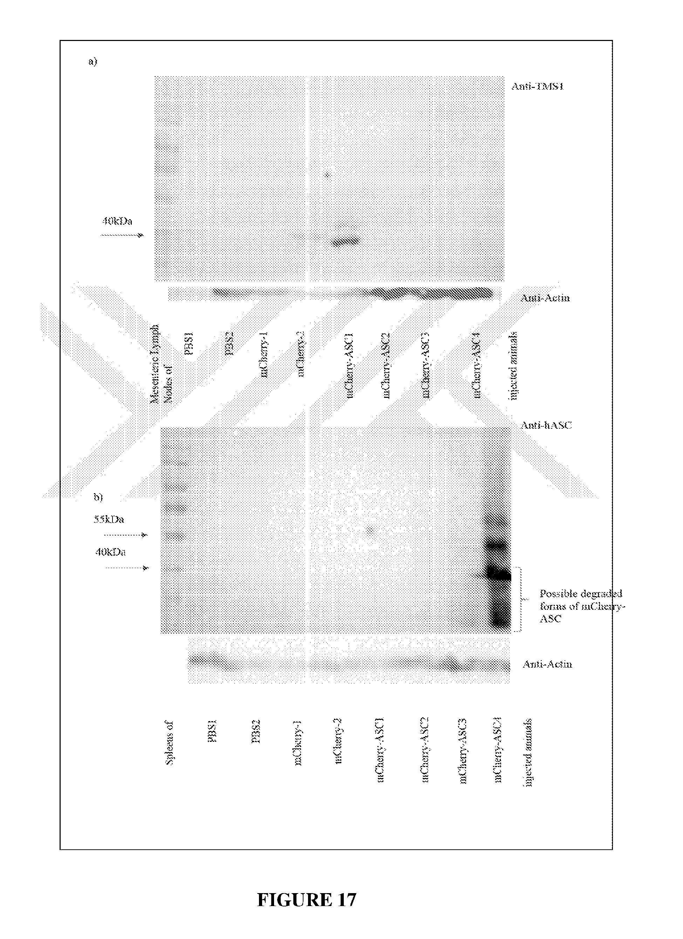

Vaccines are generally injected subcutaneously, intramuscularly or intraperitoneally to stimulate immunity. The success of the vaccines are increased by draining of the antigen to the lymph nodes where APCs interact to lymphocytes (Johansen, P. and T. M. Kündig, "Intralymphatic immunotherapy and vaccination in mice.", Journal of visualized experiments : JoVE , No. 84, p. e51031, 2 2014 ; Tozuka, M., T. Oka, N. Jounai, G. Egawa, K. J. Ishii, K. Kabashima and F. Takeshita, "Efficient antigen delivery to the draining lymph nodes is a key component in the immunogenic pathway of the intradermal vaccine", Journal of Dermatological Science, Vol. 82, No. 1, pp. 38-45, 4 2016. ) Moreover, delivery of antigens from injection site to lymphoid organs by migratory DCs increase the efficiency of CTL priming (Allan, R. S., J. Waithman, S. Bedoui, C. M. Jones, J. A. Villadangos, Y. Zhan, A. M. Lew, K. Shortman, W. R. Heath and F. R. Carbone, "Migratory Dendritic Cells Transfer Antigen to a Lymph Node-Resident Dendritic Cell Population for Efficient CTL Priming", Immunity, Vol. 25, No. 1, pp. 153-162, 7 2006. ) In order to understand whether ASC specks trigger antigen drainage to secondary lymph nodes, mCherry-ASC specks were intraperitoneally injected to C57BL/6 mice and visualized with IVIS technology. Interestingly, red florescence signal from spleen and mesenteric lymph node of mCherry-ASC injected animals was recognized one day after immunization whereas there was no florescence signal in PBS and only mCherry injected animal when they are excited at 580 nm (Figure 16). Moreover, the inventors verified IVIS results by performing anti-hASC blot from mesenteric lymph node and spleen tissue after sacrificing animals two days after injection (Figure 17).

The details of the tests are explained in the experiments part of the current description. Below short information about these experiments and their results are explained.

Firstly, tOVA-ASC protein (a fusion protein of truncated form of OVA including aminoacids sequence between 239 and 386, and human ASC protein) is produced by p-C3-d (1-238) OVA-ASC plasmids.

Truncated form of OVA includes both T cell epitopes of OVA; OVA peptide between 257 and 268 amino acids and OVA peptide between 323-339 amino acids are MHC Class I and MHC Class II epitopes, respectively (Lipford, G. B., M. Hoffman, H. Wagner and K. Heeg, "Primary in vivo responses to ovalbumin. Probing the predictive value of the Kb binding motif.", Journal of immunology (Baltimore, Md. : 1950), Vol. 150, No. 4, pp. 1212-22, 2 1993; McFarland, B. J., A. J. Sant, T. P. Lybrand and C. Beeson, "Ovalbumin (323-339) peptide binds to the major histocompatibility complex class II I-A(d) protein using two functionally distinct registers.", Biochemistry, Vol. 38, No. 50, pp. 16663-70, 12 1999). tOVA-ASC proteins are used to produce tOVA-ASC specks.

Additionally, tOVA+ASC specks are produced by loading tOVA proteins to ASC specks which are then used for the investigation of the adjuvant effect of ASC specks in cancer immunotherapy.

Hereby, it is important to note that there are different ways to load a tumor antigen to an ASC speck. A reference is given to the published patent application WO 2013/160807 (A method for antigen delivery) for some of the methods to load a bioactive molecule in general, according to the present invention specifically a tumor antigen, to an ASC speck.

For instance a tumor antigen can be loaded to an ASC speck carrier via hydrophobic interactions or by forming a fusion protein of at least one antigen with ASC proteins forming the ASC speck carrier.

Antigens with hydrophilic properties cannot be carried with ASC speck carriers via hydrophobic interactions alone. Accordingly, in one embodiment antigens with hydrophilic properties can be loaded to the ASC speck carrier via hydrophobic interactions by creating a fusion protein of antigen with a peptide comprising at least 13 amino acids long and hydrophobic sequence.

Secondly, mCherry-ASC specks are produced and purified according to the method described in WO 2013/160807. mCherry is a red fluorescent protein used as a tracker for imaging systems.

The experiments conducted with above mentioned ASC specks have shown that purified ASC specks are engulfed and degraded by macrophages. Moreover, it has been documented that stimulation of THP-1 macrophages with purified ASC specks induces secretion of inflammatory cytokines; IL-1β, IL-6 and TNF-α. Furthermore, it has been shown that tOVA-ASC speck injections cause secretion of type I and type II cytokines from splenocytes of the immunized wild type C57BL/6 mice. Finally, the inventors of the present invention have demonstrated that tOVA-ASC injections cause complete eradication or reduction in size of the EG7-OVA tumor in C57BL/6 mice.

As mentioned above Chicken Ovalbumin Protein (OVA) is used as a model antigen for the purposes of the present invention. p-C3-d (1-238)OVA-ASC plasmids are cloned for the production of tOVA-ASC (fusion protein of truncated form of OVA, chicken egg albumin protein, including aminoacids sequence between 239 and 386, and human ASC protein).

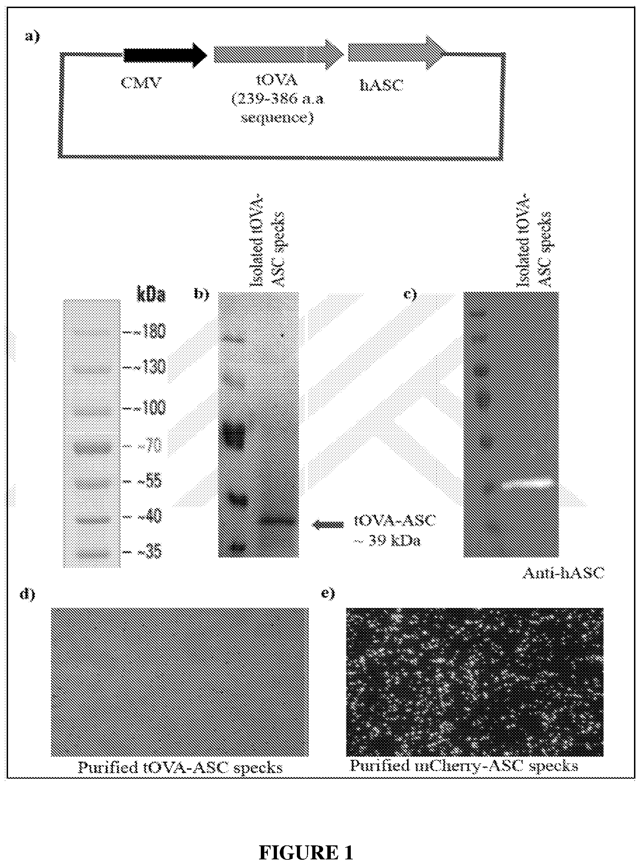

To test immune stimulatory effect of ASC specks in vivo and on THP-1 cell line, mCherry-ASC specks and tOVA-ASC specks were produced in HEK293 FT cell line. To determine the purity and concentration of tOVA-ASC speck, purified specks and BSA with pre-determined concentration were loaded into SDS-PAGE and stained with Coomasie blue as seen in Figure 1. Anti-hASC blot of isolated protein was done to understand whether the purified protein is tOVA-ASC.

To investigate the effect of ASC specks on antigen uptake, PMA differentiated THP-1 macrophages were treated with 10 ug/ml, 25 ug/ml, 50 ug/ml and 100 ug/ml of mCherry-ASC specks. PBS and 50 ug/ml of commercial mCherry protein were used as negative controls. 12 hours after treatments, no mCherry signal was observed in wells treated with PBS and mCherry protein but mCherry-ASC specks treated cells engulfed mCherry-ASC specks as seen in Figure 2. The hASC blot of the treated cells shows also engulfed and degraded mCherry-ASC protein.

To confirm florescent data and quantify it, the same experiment was repeated and mCherry positive cells were counted with flow cytometry 12 hours after treatment. Number of mCherry positive cells increases with increasing concentration of mCherry-ASC specks in treatments. With these experiments, it has been showed that ASC specks enhances engulfment of mCherry protein by THP-1 macrophages.

Degradation of engulfed mCherry-ASC specks by THP-1 macrophages were detected under florescent microscope (Figure 3). 4 hours after mCherry-ASC treatment, when 40X objective of the florescent microscope was used, red florescent signals came from cytoplasm of the cells and it was any more spread through cytoplasm of the cells rather than speck like structure. Expression levels of NLRP3 ASC and pro-caspase-1 (pro-inflammatory proteins) were determined with RT-qPCR after treatments with 10 µg mCherry-ASC specks, 10 µg mCherry and 10 µg PBS. HPRT, housekeeping gene, was used as control for qPCR and all gene expressions were normalized according to HPRT expression. Expression levels of NLRP3, pro-caspase-1 and ASC significantly increased after mCherry-ASC treatment compared to mCherry treatment as seen in Figure 5.

To understand whether ASC speck causes a pro-inflammatory response after engulfment, secreted cytokines were determined from the medium of PMA differentiated THP-1 macrophages 12 and 24 hours after mCherry-ASC treatment. PBS, mock purification sample and commercial mCherry protein were used as negative controls. mCherry-ASC speck treatment increased secretion of IL-1 β and TNF- α levels in time and concentration dependent manner while mCherry and mock purification sample treated cells secreted the cytokines in basal level as seen in Figure 5.

Consequently, it has been speculated that engulfment of the peptides is enhanced and inflammatory response can be formed even though peptide used is not antigenic by ASC specks in THP-1 macrophages.

Furthermore, to see whether tOVA-ASC specks are also engulfed by APCs, PMA differentiated THP-1 macrophages were treated with 10 ug/ml tOVA-ASC specks and immunocytochemistry assay was done with anti-hASC antibody 12 hours after treatment. 10 ug/ml OVA and PBS were used as negative controls. Alexa-488 conjugated anti-mouse IgG were used as the secondary antibody. Poor green florescent signals in the Figure 6 show endogenous ASC since it is normally found in the cells as distributed through cytoplasm but after activation of inflammation it forms a speck like structure. Punctiform strong green florescent signals in the Figure 6 show the engulfment of tOVA-ASC specks by THP-1 macrophages.

To be sure about all of the specks are not endogenously formed, Western blot analysis with anti-TMS1 (hASC antibody produced in rabbit) was done. For Western blot analysis, THP-1 macrophages were treated with 2.5 ug/ml, 5 ug/ml and 10 ug/ml of tOVA-ASC. 10 ug/ml OVA and PBS were used again as negative controls. 12 hours after treatment, wells were washed three times with PBS to remove tOVA-ASC specks which are not engulfed yet and Western blot analysis was done with anti-TMS1 and anti-OVA.

As it can be seen in Figure 6, the size of the bands is about 39 kDa which shows the punctiform green florescent signals came from tOVA-ASC specks. Also, OVA can be engulfed by macrophages without need of an adjuvant or vaccine carrier (3_{rd} lane in Figure 6).

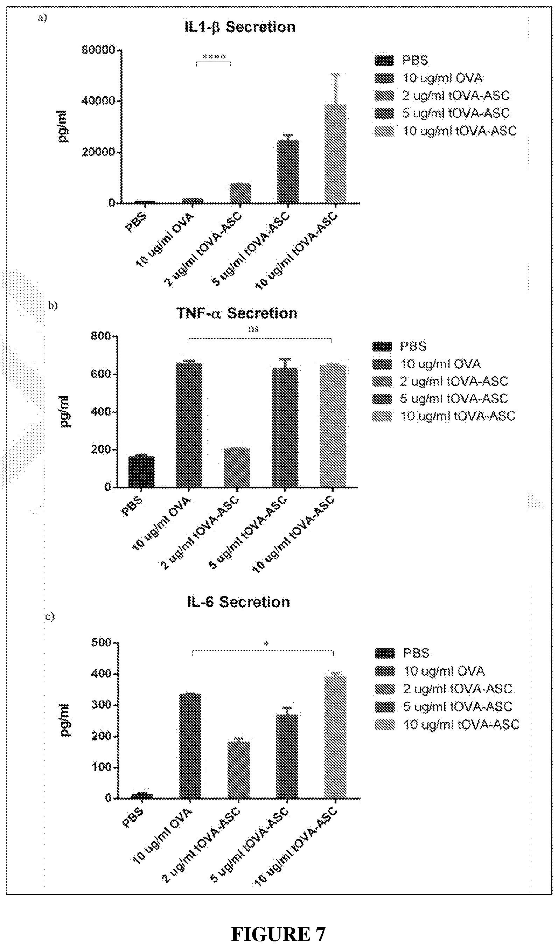

Medium of the cells were also collected to determine secreted IL-1β, IL-6 and TNF- α levels by ELISA. tOVA-ASC speck treatment significantly increased IL-1β and IL-6 secretion (Figure 7). Secretion of IL-1β from THP-1 macrophages was higher than at each concentration of tOVA-ASC treatment than 10 µg OVA treatment and it increased in concentration dependent manner. IL-6 secretion levels after tOVA-ASC treatment increased in a dose dependent manner and it is higher in 10 µg /ml tOVA-ASC treatment than 10 µg /ml OVA treatment alone. The TNF- α levels are also increased with increasing concentration of tOVA-ASC amount. However, there is no significant difference between 10 µg /ml OVA and 10 µg /ml tOVA-ASC treatment in TNF- α secretions.

Chicken ovalbumin synthesizing C57BL/6 mice thymoma cell line, EG7-OVA, is one of the mostly used tumor model for antigen specific tumor vaccine studies. They express OVA when they are treated with G418. Before inoculation to mice, OVA expression of EG7-OVA cell line was shown with anti-OVA blot as shown in Figure 9.

To form tumors in 8-10 weeks old C57BL/6 mice, 2.5 million EG7-OVA cell were inoculated into each dorsal flank of the mice. 12 days after inoculation, 18 tumors reached a palpable size and intraperitoneal injections of 100 µg of tOVA-ASC in 300 µl PBS were started as shown in Figure 9. 50 µg OVA and PBS were used as negative controls. Two sizes of the tumors were determined every second days with a two digit caliper after first immunization.

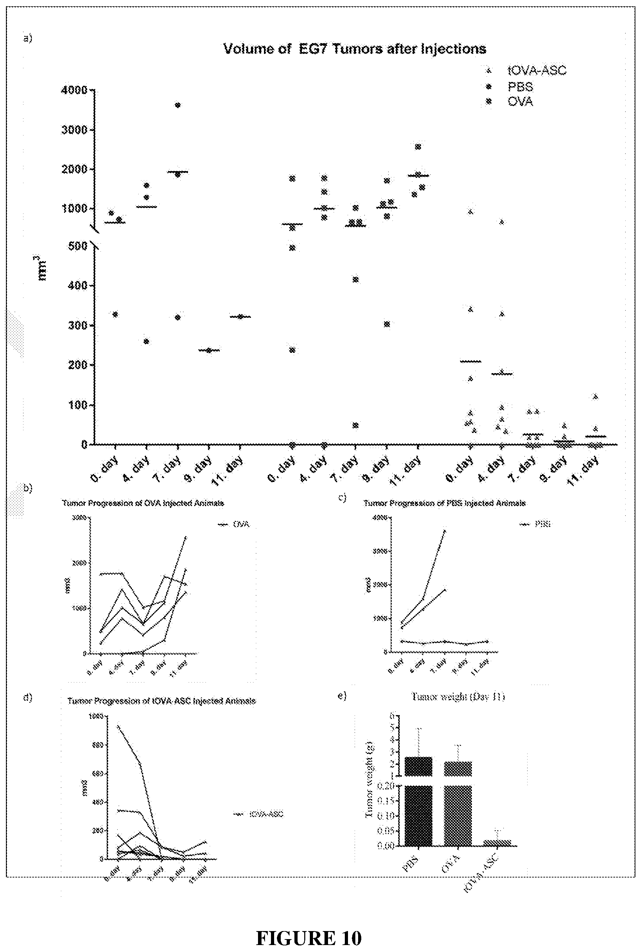

One tumor in tOVA-ASC injected animals was completely eradicated at day 4 after first injection. Three tumors more in tOVA-ASC injected animals were completely eradicated at day 7 after first injections and one more tumor formed in one of the OVA injected animals. 1 week after first immunization, booster injections were repeated as in first one. One day after second immunization, one of the PBS injected animals died possibly because of the size of tumors it had. Two tumors more in tOVA-ASC injected animals were completely eradicated at day 2 after second injections as seen in Figure 10.

Experiment was ended to do further analysis with tumors of tOVA-ASC injected animals since only 2 tumors remained after second injections at day 4 after second immunization. Progression of each tumor in each group was shown. All animals were sacrificed. No tumor tissue was recognized in tOVA-ASC injected animals whom tumors had already been eradicated. One of OVA injected animals had two separate tumor tissues in the same dorsal flank. Average volumes of excised tumors of PBS, OVA and tOVA-ASC injected are 1935 mm³, 1690 mm³ and 62 mm³, respectively as shown in Figure 11. Average tumor weights of PBS, OVA and tOVA-ASC injected tumors were 2.5 g, 2.11 g and 0.07 g, respectively as shown in Figure 11.

Consequently, two shots of tOVA-ASC specks cause reduction in size of each tumor and complete eradication of 6 out of 8 EG7-OVA tumors as tumors of PBS and OVA injected animals continue to grow in dorsal flank of C57BL/6 mice as shown in Figure 10.

Since, anti-tumor effect of tOVA-ASC specks can be caused by antibody response against OVA, OVA specific IgG levels of animals are determined by ELISA. Since one animal in PBS group already died before sacrificed, the blood of the animal cannot be taken and further experiments are conducted with only one animal for PBS group. Consequently, tOVA-ASC injected animals had more OVA IgG response than PBS and OVA injected animals as it can be seen in Figure 12.

Further tests have been conducted to investigate the adjuvant effect of ASC specks.

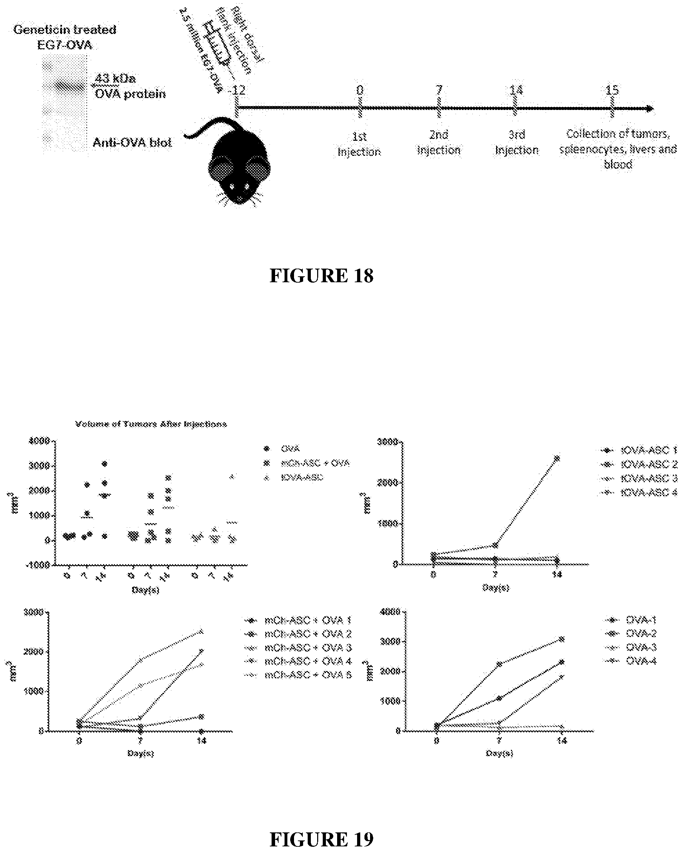

There were 4 groups including PBS, OVA, tOVA-ASC and mCh-ASC+OVA experimental groups. Only PBS, only 100 µg of OVA, only 100 µg of tOVA-ASC and 100 µg of mCh-ASC and 100 µg of OVA together were injected intraperitoneally to each group of mice. PBS was a negative control group because both OVA and ASC specks were suspended in PBS. To investigate the adjuvant effect of ASC specks, OVA injections were performed to see whether OVA immunization alone was sufficient for a reduction in tumor size. Also, mCh-ASC with OVA group was a model to see an adjuvant property of ASC specks comparing to OVA group. In this experiment, tOVA-ASC group was the model group to see the carrier ability of ASC specks as antigen-specific anti-tumor vaccine. Vaccination timeline for immunization of mice that had EG7-OVA tumors can be seen in Figure 18.

14 days after inoculation of EG7-OVA cells, tumor formation reached to palpable size to start immunizations. As seen in Figure 19, 7 days after the first immunization, although PBS, OVA and mCh-ASC+OVA groups had an increase in tumor size, tOVA-ASC specks immunized mice had almost no progression in tumor sizes. Then, second doses were injected to each group.

7 days after the second immunization, one of mCh- ASC+OVA immunized mice and three of tOVA-ASC immunized mice had completely eradicated tumors. One of the tumors from the mice that only OVA immunized remained at the same size after 2 injections. One of mCh-ASC+OVA immunized mice stayed with stable tumor size after 2 injections. After last immunization, all animals were sacrificed. Lastly, one of the tOVA-ASC immunized mice showed a tumor grown pattern after the injection.

Antibodies have been in clinical trials for targeting the tumor cells. These antibodies bind to antigens on tumor cells and induce direct cytolysis or antibody promoted phagocytosis, resulting in the processing of antigens and presentation via MHC Class I or II molecules on APCs. This circumstance induces anti-tumor immunity of host by either the production of the tumor-directed antibodies and/or cytotoxic T cells in the host. Anti-tumor effect of tOVA-ASC specks was observed so, this anti-tumor activity could be caused by antibody promoted antitumor immunity. To show antigen-specific antibody production in host, ELISA is performed to see OVA-specific IgG response against EG7-OVA tumors.

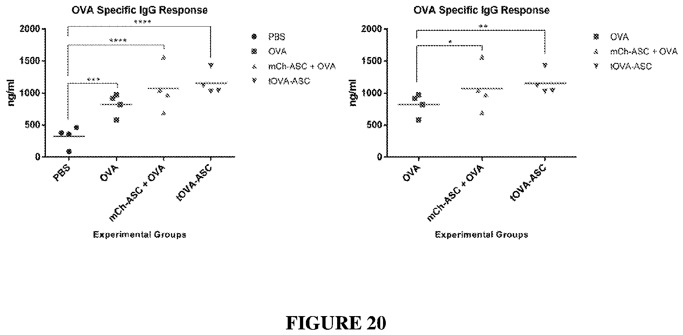

As seen in Figure 20, PBS was the negative control group, and comparing to other groups with PBS, the increase in the OVA-specific IgG levels were obtained for OVA, mCh-ASC+OVA and tOVA-ASC groups. When OVA including groups were compared to each other, there was a significant higher IgG response for mCh- ASC+OVA group comparing to the OVA group. Also, there was a significant higher IgG response for tOVA-ASC group comparing to the OVA group. This showed that ASC specks could have anti-tumor activity by boosting the immune system as an adjuvant and by carrying antigens to induce anti-tumor immunity in the host.

Lastly, it has been shown that intraperitoneally injected mCherry-ASC specks goes to secondary lymph nodes which is important for the triggering of the adaptive immune system.

Since secondary lymph nodes are the main location for T cell responses, the inventor of the present invention checked whether intraperitoneally injected mCherry-ASC specks can be transported into mesenteric lymph node and spleen by IVIS and Western blot analysis. For this purpose, 10 ug mCherry-ASC specks in 200 ul of PBS intraperitoneally injected to eight 16-18 weeks old C57BL/6 mice. As negative controls, four and eight animals were injected with PBS and commercial mCherry protein, respectively. One, two and ten day after injections, animals were monitored under IVIS machine. At day 2, half of the animals were sacrificed and their spleen, mesenteric lymph nodes, livers and brain were collected for western blot analysis. The other half was sacrificed at day 10 and same tissues were collected.

As it can be seen in Figure 16, yellow indicates high intensity of red fluorescent signal in IVIS and abdominal and genital regions of C57BL/6 animals give background florescence when animals excited at 56-580 nm wavelength. However, intense florescence was taken one of mesenteric lymph node region of the mCherry-ASC injected animal one day after the injections. At day 10, florescence intensity was almost the same for each animal.

To support IVIS data, all taken tissues were blotted with anti-hASC produced in rabbit to detect injected mCherry-ASC specks. As it can be seen in Figure 17 mCherry-ASC protein was detected in mesenteric lymph of node of one animals and spleen of another one. Moreover, degradation of mCherry-ASC protein can be seen in lower bands of anti-hASC blotting of spleens.

Accordingly, in one embodiment of the present invention a tumor antigen and ASC specks (which are formed by ASC proteins) are used in the treatment of cancer as the experiments have demonstrated their ability to boost the immune system against tumor cells.

In another embodiment a composition comprising a tumor antigen and an ASC speck formed by ASC proteins for use in a method of cancer therapy is provided wherein said method comprises inducing and/or stimulating an immune response against a cancer disease in a mammal, wherein said induction or stimulation causes a decrease in cancer progression or growth arrest of cancer cells.

In another embodiment a composition comprising a tumor antigen and an ASC speck molecule formed by ASC proteins is provided for use in the cancer treatment.

In another embodiment a composition of ASC speck for use in cancer therapy is provided wherein the composition contains a tumor antigen which is carried by the ASC speck by forming hydrophobic interactions with the ASC proteins forming the ASC speck.

According to one embodiment of the present invention the composition comprises an ASC speck which is formed by fusion proteins of a tumor antigen and at least one ASC protein. Said composition is used in the treatment of cancer, particularly in the treatment of tumors.

The above mentioned fusion protein can be formed by a tumor antigen being fused to the ASC protein at the N-terminus or C-terminus.

In another embodiment of the present invention the composition comprises a tumor antigen being carried inside the ASC speck, preferably by forming hydrophobic interactions with the ASC proteins forming the ASC speck.

The present invention is directed to the treatment of any type of cancer, preferably a tumor forming cancer and more preferably thymus cancer.

According to one embodiment, the compositions of the present invention are used in the cancer immunotherapy, whereby their administration to a mammal in need thereof causes a decrease in tumor progression or a decrease in tumor size or a complete eradication of the tumors.

In another embodiment of the present invention a method of treating cancer is provided, comprising the step of administering a therapeutically effective amount of a composition comprising an ASC speck to the mammal in need thereof. Said composition comprises a tumor antigen; preferably the tumor antigen is carried by the ASC speck by forming a fusion protein with the ASC proteins forming the ASC speck.

In another embodiment a composition comprising a tumor antigen bound ASC speck carrier is provided wherein the ASC speck carrier is formed by the oligomerization of fusion proteins of ASC protein and tumor antigen.

Examples of the tumor antigens that can be used for the present invention are HER2+ (a membrane-bound protein) for breast cancer, PSA (a glycoprotein with the size of 34kDa) for prostate cancer, MAGE (melanoma associated antigen) for melanoma cancer, MUC1 (a transmembrane glycoprotein) for ductal glycoprotein or neoantigen. These are given just as examples, any other tumor associated antigen can be used for the purposes of the present invention.

In yet another embodiment of the present invention a composition comprising a tumor antigen bound ASC speck carrier is used in the treatment of thymus tumors wherein ASC speck is formed by the fusion proteins of ASC and tumor antigens.

### EXPERIMENTS

### MATERIALS:

Details of the materials used in the experiments are shown below.

**Table 1. Cell Lines**

| **.Cell Line** | **Main Source** | **Catalog Number** |
|---|---|---|
| HEK293FT | Invitrogen, USA | R700-07 |
| THP-1 | ATCC, USA | TIB-202 |
| THP-1 eGFP-ASC | THP-1 cell line | - |
| EG7-OVA | ATCC, USA | CRL-2113 |

**Table 2. Cell Culture Medium**

| **Medium** | **Catalog Number** | **Source** |
|---|---|---|
| DMEM, high glucose, pyruvate | 41966029 | Gibco, Thermo Fischer Scientific, USA |
| RPMI MEDIUM 1640 | 21875034 | Gibco, Thermo Fischer Scientific, USA |
| FBS (Fetal Bovine Serum) | 10270106 | Gibco, Thermo Fischer Scientific, USA |
| Penicillin-Streptomycin | 15140122 | Gibco, Thermo Fischer Scientific, USA |
| MEM NEAA- 100X | 11140050 | Gibco, Thermo Fischer Scientific, USA |
| 2-mercaptoethanol | 21985-023 | Gibco, Lifetechnologies, USA |
| G418 Geneticin | 1150MG250 | Neofroxx, Germany |

**Table 3. Cell Culture Solutions**

| | |
|---|---|
| 10X PBS | 1.37 M NaCl |
| | 27 mM KCl |
| | 15 mM KH₂PO₄ |
| | 81 mM Na₂HPO₄ |
| | pH:7,4 |
| ACK Lysis Buffer | 154 mM NaH₄Cl |
| | 10 mM KHCO₃ |
| | 0.1 mM Na₂EDTA |
| Freezing Medium | 10% DMSO (A3672, AppliChem, Germany) |
| | 90% Growth medium |
| 0,05 % Trypsin | 0,53 mM EDTA |
| | 0,5 g/L Trypsin |
| | 8 g/L NaCl |
| | 0,4 g/L KCl |
| | 0,06 g/L KH2PO4 |
| | 1 g/L D-Glucose |
| | 0,048 g/L Na4HPO4 |
| | 0,35 g/L NaHCO3 pH:7,5-8 |
| 2M CaCl2 | 110,98 g in 500 ml ddH2O |
| 2X HBS | 25 ml Hepes |
| | 7,3 g NaCl |
| | 0,1 g Na2 HPO4 |
| | for 500 ml, pH:7,05 |

**Table 4. Western Blot Solutions**

| | |
|---|---|
| NP40 Lysis Buffer | 150 mM NaCl, 0,2% NP-40 |
| | 50 mM Tris, pH:8 |
| 30:0,8 Acrylamide:Bisacrylamide | 30 g Acrylamide |
| | 0,8 g Biasacrylamide in 100 mL ddH₂O |
| 4% Stacking gel | 66 mL 30:0,8 Acrylamide:Bisacrylamide |
| | 126 mL 0,5 M Tris-HCl, ph:6,8 |
| | 5 mL 10% SDS in 500 mL ddH₂O |
| 15% Resolving Gel | 500 mL 30:0,8 Acrylamide:Bisacrylamide |
| | 200 mL 1,875 M Tris, ph:8,8 |
| | 10 mL 10% SDS in 1 L ddH₂O |
| 5X Laemmli Buffer | 1,5 M Tris-HCl, ph:6,8 10 ml Glycerol |
| | 5 ml beta-mercaptoethanol |
| | 2 g SDS |
| | 1ml 1% bromophenol blue in 20 ml ddH₂O |
| APS | Applichem, Germany |
| TEMED | Sigma, Germany |
| Running Buffer (10 X Tris-Glycine) | 30 g Tris base |
| | 144 g Glycine |
| | In 1 Liter dH₂O |
| Semi-Dry Transfer Buffer | 2,93 g Glycine (39 mM) |
| | 5,81 g Tris-base (47 mM) |
| | 6,25 ml 10% SDS |
| | In 1 Liter dH₂O |
| | Adjusted pH: 9.2 |
| Methanol | Merck, USA |
| Bovine serum albumin (BSA) fraction V | Roche, Germany |
| 10% BSA | 10% BSA in TBS-T |
| 10X TBS Buffer | 1.5 M NaCl |
| | 0.5 M Tris, pH:7.5 |
| Tween-20 | |
| TBS-T | 100 ml 10X TBS Buffer |
| | 0.1% Tween-20 |
| Sodium azide | Roche, Germany |
| Prestained Protein Ladder | PageRuler, Thermo Fischer Scientific, USA |
| Enhanced Chemiluminescence Substrate (ECL] | Advansta Inc., USA |
| Coomassie Blue Staining Solution | 0.1% Coomassie Blue |
| | 10% Acetic acid |
| | 50% Methanol |
| | In dH₂O |
| Coomassie Blue Destaining Solution | 10% Acetic acid |
| | 40% Methanol |
| | In dH₂O |
| Elution Bu_er for SDS-PAGE | 50 mM Tris-HCl |
| | 150 mM NaCl |
| | 0.1 mM EDTA |
| | Adjusted pH: 7.5 |

**Table 5. ELISA Solutions**

| **Solution** | **Ingredients** |
|---|---|
| Wash Buffer | 0.05% Tween 20 in PBS, pH 7.2-7.4 |
| Reagent Diluent | Filtered 1%BSA in PBS, pH 7.2-7.4, |
| Substrate Solution | 1:1 mixture of Color Reagent A (H₂O₂) and Color Reagent B (Tetramethylbenzidine). |
| Stop Solution | 2N H₂SO₄ |

**Table 6. Antibodies**

| **Antibody** | **Conjugate** | **Catalog number** | **Catalog number** |
|---|---|---|---|
| anti-rabbit IgG | HRP | 7074S | Cell Signaling Technology, Netherlands |
| anti-mouse IgG | HRP | 7076S | Cell Signaling Technology, Netherlands |
| anti-hASC | - | - | Kindly provided by Prof. Mosumoto, Japan |
| anti- *β-*Actin | - | 4970S | Cell Signaling Technology, Netherlands |
| anti-TMS1 | - | ab155970 | Abcam, UK |
| anti-OVA | - | ab17293 | Abcam, UK |
| anti-mCherry | - | ab125096 | Abcam, UK |
| anti-CD8-*α* | APC | 100711 | Biolgend, USA |
| anti-CD16/CD32 | - | 553141 | BD Biosciences, USA |
| anti-CD8-α | Alexa647 | - | Kindly provided by Prof. Batu Erman, Turkey |
| anti-B220 | PE | - | Kindly provided by Prof. Batu Erman, Turkey |
| anti-CD4 | PE | - | Kindly provided by Prof. Batu Erman, Turkey |
| ant-H57 | FITC | - | Kindly provided by Prof. Batu Erman, Turkey |
| Leu4 | FITC | - | Kindly provided by Prof. Batu Erman, Turkey |
| Leu4 | PE | - | Kindly provided by Prof. Batu Erman, Turkey |

**Table 7. Plasmids**

| | |
|---|---|
| p-C3-d(1-238)OVA-ASC | AKIL, Turkey |
| p-C3-mCherry-ASC | AKIL, Turkey |

**Table 8. Injection Ingredients**

| | | |
|---|---|---|
| Aluminum potassium sulfate dodecahydrate | 237086 | Sigma, Germany |
| Complete Freunds Adjuvant (CFA) | 344289 | Calbiochem, Germany |
| Incomplete Freunds Adjuvant (ICFA) | SC37726 | Santa Cruz, USA |
| Chicken Ovalbumin | A5503 | Sigma, Germany |
| mCherry | 4993-100 | Bio Vision, USA |

**Table 9. Kits**

| | | |
|---|---|---|
| Mouse IL-4 DuoSet ELISA | DY404-05 | R&D Systems, USA |
| Mouse IFN-gamma DuoSet ELISA | DY485-05 | R&D Systems, USA |
| Mouse TNF-alpha DuoSet ELISA | DY410-05 | R&D Systems, USA |
| Human IL-1 beta/IL-1F2 DuoSet ELISA | DY201 | R&D Systems, USA |
| Human IL-6 DuoSet ELISA | DY206 | R&D Systems, USA |
| Human TNF-alpha DuoSet ELISA | DY210 | R&D Systems, USA |
| NucleoBond Xtra Midi | 740410.50 | Macherey-Nagel, Germany |
| NucloeSpin RNA | 740955.50 | Macherey-Nagel, Germany |

### METHODS:

### 1. Purification of tOVA-ASC and mCherry-ASC Specks

### 1.1. Plasmid DNA Amplification and Isolation

p-C3-d (1-238) OVA-ASC and p-C3-mCherry-ASC plasmids are cloned and used for this study. Midi- and maxi preparations were done by using MACHEREY- NEGEL (MN) kits as manufacturer suggests. Bacterial cultures were grown in 200 ml and 400 ml LB supplemented with kanamycin for midi and maxi preparations, respectively.

### 1.2. Maintenance of HEK293FT Cell Line

HEK293FT cell line was maintained in DMEM supplemented with 10% FBS, MEM-NEA, 100 U/ml penicillin and 100 µg/ml streptomycin. When the cells reached 90% confluency, the plates were washed with PBS and trypsinized at 37°C for 2 min to subculture.

### 1.3. Calcium-Phosphate Transfection

HEK293FT cell lines was transfected easily by calcium phosphate transfection method which causes formation of insoluble precipitates of plasmid DNA and calcium phosphate. These precipitates are engulfed by HEK293FT cells. 14 million cells were seeded into 150 mm cell culture plate. Plasmid concentration was adjusted by diluting in ddH2O according to experiment and 2M CaCl2 was added drop by drop. After 5 min, 2X HBS was added and bubbles were formed. After 5 min, plasmid and calcium phosphate mix was added drop by drop onto HEK293FT cells during subculture.

**Table 10. Transfection Mixture.**

| Plate Type | DNA | ddH₂O | 2M CaCl₂ | 2X HBS |
|---|---|---|---|---|
| 150 mm Plate | 30-40 *µ*g | 1317 *µ*l | 183 *µ*l | 1.500 *µ*l |

### 1.4. Production and Isolation of ASC Specks

14 million HEK293FT cells were counted and plated to each of six 150 mm cell culture plates. (pC3-(d1-235) OVA-ASC) plasmid DNA was transfected via calcium phosphate transfection method. 48 hours after transfection, mediums of cell were aspirated and 3 ml PBS was added. Cells were detached from surface by scraping and was taken into 50 ml falcon. Cells were lysed 5 times via sonication for 30% of 5 seconds under 50% power. Lysed cells were centrifuged at 2400 g with Beckman table centrifuge at 4°C. After centrifugation pellet was dissolved until filaments were easily detectable by vortex. Large fragments of cells was settled down via gravity sedimentation for 5 minutes on ice. Supernatant was carefully transferred to a new 50 ml falcon and centrifuged at 300 g for 5 minutes. Supernatant was aspirated and dissolve pellet in 10 ml PBS. Pellet was again dissolved by vortex until filaments were visible. Filaments were again settled down via gravity sedimentation for 5 minutes.

Supernatant was taken into a new falcon and centrifuged at 300 g. Last vortex, gravity sedimentation and centrifugation were repeated until there was no more filament after vortex. Last sample was filtered through 1.2 or 5 µm syringe filter. 3-4 ml of sample was passed through for each filter until backpressure was sensed. 2-3 ml of PBS was applied to filter in reverse direction with 200 µl micropipette to rescue ASC specks into 15 ml falcon. Reverse filtrate was centrifuged at 3900 g for 1 hour. Pellet was dissolved in 500 µl of PBS. ASC specks in PBS were kept at +4°C. Purity of ASC specks was checked via SDS-PAGE and Coomasie blue staining. All steps except sonication was done in cell culture hood.

### 1.5. Determination of Purity and Amount of Isolated ASC Specks with SDS-PAGE and Coomasie Blue Staining

40 µl of isolated ASC speck and BSA at different concentrations mixed with 10 µl of 5X Leamli buffer in 1.5 ml tube, separately. They were boiled at 95°C for 10 min and loaded with prestained protein ladder on 12% SDS-PAGE gel. The samples were run as described in protein gel electrophoresis part. The gel was stained with Coomasie blue in a plastic container for 1 hour and destained with Coomasie destaining solution. Photograph of the gel was taken and the concentration of ASC specks was calculated according to standard curve derived from BSA concentration with ImageJ software.

### 2. Effect of ASC Specks on THP-1 Macrophages

### 2.1. Maintenance of THP-1 Monocytic Cell Line

THP-1is a monocytic cell line derived from a leukemia patient. THP-1 cells were provided and constitutively eGFP-ASC synthesizing THP-1 eGFP-ASC stable cell line was derived from THP-1 monocytes via lentiviral transduction of pLenti-Efla-EGFP-ASC. The cell lines were maintained in RPMI 1640 supplemented with 10% FBS, MEM-NEA, 100 U/ml penicillin and 100 µg/ml streptomycin.

### 2.2. Differentiation of THP-1 Monocytes to THP-1 Macrophages

1.5x106 THP-1 monocyte cells were differentiated to THP-1 macrophages in each well of 6 well plate with 100 nm/ml PMA containing RPMI 1640 for 9 hours. Then, media of the cells were changed with PMA free RPMI 1640.

### 2.3. Treatment of THP-1 Macrophages with ASC Specks

48 hours after differentiation, mCherry-ASC or tOVA-ASC specks were given onto 1 ml of media of the cells at different concentrations to see the effect of ASC specks on macrophages. Media of the cells were collected and kept at -20 C for further cytokine ELISA assay at different time points. Preparing of THP-1 Macrophages for Flow Cytometry Analysis 12 hours after ASC specks treatment, cells were incubated with PBS-EDTA on ice for 30 min. Then, cells detached from cell culture plate by scraping gently and taken into 15 ml centrifuge tube. The cells were centrifuged at 300 g for 5 minutes and washed twice with FACS buffer containing human serum. Their cell surface proteins were stained and analyzed as described in Cell Surface Staining and Flow Cytometry Analysis part.

### 2.4. Immunocytochemistry of THP-1 Macrophages

1.6x10⁵ THP-1 monocyte was differentiated into THP-1 macrophages in 48 well plate and treated with ASC specks. 12 hours after ASC treatment, medium of the cells was aspirated and wells washed three times with PBS. Then, cells were fixed with 4% PFA for 15 min at room temperature. After wash of the cells 3 times with PBS, they were permeabilized with PBS including 0.25% Triton- X100 detergent. Then, cells were blocked with blocking buffer (5% BSA (w/v) in PBS-T) for 30 min at room temperature. Then, cells were incubated with 1/50 diluted anti-hASC antibody in blocking buffer for 2 hours at room temperature. Cells again washed with PBS and incubated with 1/500 diluted Alexa-488 fluorophore conjugated anti-mouse IgG for 1 hour at room temperature. After incubation of secondary antibody, cells were washed and visualized under fluorescent microscope.

### 2.5. Cytokine ELISA

Cytokine levels in medium of the cells was determined with sandwich ELISA. Briefly, ELISA plates were coated with capture antibody in PBS at a concentration that manufacturer suggests and incubated overnight at RT. Three wells of 96 well plate was coated for each supernatant sample and three wells were coated with only PBS as a negative control. After overnight incubation, the plates were washed three times with 400 µl of PBS-T and blocked with 200 µl of reagent diluent (1% BSA in PBS) for one hour at RT to prevent non-specific binding. During blocking of the plate, supernatant of the cells was diluted in reagent diluent as final OD value is below than 2.00. After blocking, the plates were washed three times with PBS-T and 100 µl of diluted samples and cytokine standards were added to wells and incubate at room temperature for 2 hours. Then, the plates were again washed three times with PBS-T and incubated with detection antibody in 100 µl of reagent diluent at a concentration that manufacturer suggests for two hours at RT.

After washing of the plates, 100 µl of 1:50 diluted HRP in reagent diluent was added onto wells and incubated for one hour at RT. Then, the plates were again washed and 100 µl of 1:1 mixed ELISA Substrate A and ELISA Substrate B added onto wells and color started to turn blue. 20 min after substrate addition, reaction was ended with 1N sulfuric acid and color changed from blue to yellow. OD value of wells was calculated with microplate reader at 450 nm and 540 nm and extracting absorbance at 540 from absorbance 450. Standard curve was drawn according to absorbance value and concentration of the standard with SoftMaxPro software and cytokine levels in supernatant were determined from standard curve.

### 2.6. Determination of Expression Pattern of Pro-Inflammatory Proteins in THP-1 Macrophages after ASC Speck Treatment with RT-qPCR

Expression pattern of the proteins in mRNA level was determined with real time quantitative PCR in PMA differentiated THP-1 cells after ASC speck treatments. THP-1 macrophages were treated with 10, 25, 50 and 100 µg of mCherry-ASC specks for 12 hours. Only PBS and 50 µg mCherry protein were used as control.

### 2.6.1. RNA Extraction.

Cells were washed 3 times with PBS and collected via scraping and RNAs were extracted with MN RNA extraction kit as manufacturer suggests. Briefly, cells were lysed with lysis buffer and lysates were bound to column after mixing with 70% ethanol. After several wash steps, columns were treated with DNase I in DNase incubation buffer for 15 min at RT. Columns were again washed with wash buffer and dried out. Then total RNA was isolated with 60 µl of RNase-free water. Purity and concentration of RNA was measured with Nanodrop spectrophotometer.

### 2.6.2. cDNA Synthesis.

cDNA was synthesized from total RNA by using BIOLINE SensiFAST cDNA synthesis kit as manufacturer suggests. Briefly, 500 ng total RNA was mixed with 4 µl of 5X TransAmp buffer and 1 µl of reverse transcriptase in DEPC treated H₂0 (Final volume: 20 µl). Samples were incubated at 25°C for 10 min, at 42°C for 15 °C and 85 °C for 5 min for inactivation.

### 2.6.3. Quantitative PCR.

Primers specific to each gene and HPR-T as a control were used to test relative expression levels of gene of focus after ASC speck treatments. cDNA samples were amplified with BIOLINE SensiFAST SYBR MasterMix using Exicycler 96 qPCR machine. Relative expressions of genes of interest were determined by adjusting the expression level of the gene in PBS treated cell as 1 and using 2^{-ddct} method (Livak and Schmittgen 2001).

### 3. EG7-OVA Tumor Model and tOVA-ASC Immunizations

### 3.1. Maintenance of EG7-OVA Cell Line

EG7-OVA cell line was provided. EG7-OVA is derived from C57BL/6 mice lymphoma cell line EL-4. EL4 cell line was stably transfected with pAc-neo-OVA plasmid including full form of chicken ovalbumin (OVA) and neomycin resistant gene to form EG7-OVA cell line which constitutively synthesize OVA protein as a model tumor antigen when treated with G418. The cell line was maintained in RPMI 1640 supplemented with 10% FBS, MEM-NEA, 100 U/ml penicillin and 100 µg/ml streptomycin, 2-mercaptoethanol and 0.4 mg/ml G418. Medium of the cells was changed every second days.

### 3.2. EG7-OVA Tumor Inoculation and Immunizations with OVA, PBS and tOVA-ASC

2.5 million EG7-OVA cells in 100 µl PBS were inoculated to each dorsal flank of 12 C57BL/6 mice. 18 tumors reached a palpable size (<50 mm₃) in 12 days. Intraperitoneal injections were done as follows; 100 µg of tOVA-ASC (n=10), 50 µg commercial OVA (n=5) and only PBS (n=3) in 300 µl of PBS. Two size of tumors was determined with a two digit caliper. Volume was calculated based on the calculation: 1/2((Short length^2) Long Length). Second injections were repeated as first one. One of PBS injected animals died possibly at day 20. Experiment was ended to do further analysis with tumors of tOVA-ASC injected animals since only 2 tumors remained after second injections. Blood of all animals were collected from orbital sinus. All animals were sacrificed and spleens of animals were collected. Tumors of animals were excised, volume and weight of them calculated. No tumor tissue was recognized in tOVA-ASC injected animals whom tumors had already been eradicated. Plasma of each animal was kept at -20°C. Splenocytes of animals were harvested and cultured in 96 well plate for further ex vivo recall assays. Tumors of animals were embedded in OCT for further immunohistochemistry assays. Small part from tumors of each group was split for Western blot analysis and kept at - 80 °C.

### 3.3. EG7-OVA Tumor Inoculation and Immunizations with PBS, OVA, tOVA-ASC and mCherry-ASC

Similar to the above described experiment another test has been conducted for an additional mCherry-ASC immunization. 2.5x10⁶ EG7-OVA cells in 100 µl 1X PBS were inoculated for each mice. Before inoculation, OVA expression of these cells was checked with western blot analysis using anti-OVA antibody. Cells were prepared under sterile conditions. Inoculation of these cells was performed to the right dorsal flank of each mice. When tumors reached a palpable size (≥ 50mm³) after 12-14 days, intraperitoneal injections were performed as follows; only 1X PBS, 100 µg of OVA, 100 µg of OVA with 100 µg of mCherry ASC and 100 µg of tOVA ASC in 300 µl of 1X PBS. Size of tumors was determined with a caliper. The volume of each tumor was calculated according to a formula; 1/2[(short length²).long length]. Second injections were repeated as same as the first injections.

The experiment was finished 1 week after second injections because most of the tumors of mice which immunized with tOVA ASC were eradicated. Tumors of animals were calculated via caliper. Then, all animals were sacrificed. Blood of them was collected from the hearts of them. Also, spleens and livers of them were collected for further analysis. Tumors of animals were excised and embedded in OCT. All tissues were kept on ice until the end of the experiment for the last mouse. Collected blood samples were incubated at 37°C for 1 hour then, they were centrifuged at 11000 rpm for 1 minute. Serum of blood was collected into new tubes and kept at -80°C for long term storage.

### 3.4. Immunohistochemistry of Tumors and Spleens

3.4.1. OCT Embedding. Tumor samples were put in OCT compound and kept at -20°C overnight and then kept at -80°C for further analysis.

3.4.2. Cryosection. OCT embedded tumor samples were placed to cryosection apparatus in right direction and freeze OCT without tissue was removed with a blade. Then, 10 micron tumor sections were taken onto positively charged slides using a cryostat, appropriately. Slides were dried at 55°C and kept at -80°C for further analysis.

3.4.3. Fixation and Antigen Retrieval. Slides were dried out at 55°C for two hours and tissue sections were surrounded with a hydrophobic barrier. Then, tissues were fixed with 4% PFA at RT for 15 min. PFA was removed and slides were washed three times with PBS-T in a jar on shaker. Slides were transferred to another jar filled with citrate buffer (pH: 6.0) and kept at 70°C for 30 min.

3.4.4. Antibody Staining of Cryostat Sections. After washing of the slides three times with PBS-T, they were blocked with blocking buffer (3% BSA in PBS-T) at room temperature for 30 min on a shaker. Then, slides were incubated with 1/400 diluted fluorescent labeled primary antibodies in blocking buffer overnight at 4°C. After overnight incubation, slides were washed 3 times with PBS-T for 5 min and visualized under fluorescent microscope.

### 3.5. Single Cell Preparation from EG7-OVA Tumors

Tumors were exiced and minced using scissors until all sections of tissue were processed into 1-2 mm sections. Minced samples were disaggregated against a cell strainer using a plunger from a disposable 10 ml syringe. Tumor tissue on cell strainer was washed with 3-5 mls of cold RPMI 1640. Washing step was repeated several times until observing only small fragments of connective tissue behind in the filter. The cells were washed three times with RPMI 1640 and centrifuged at RT 300 g for 5 min.

### 3.6. ELISA for OVA Specific IgG Response

OVA-specific IgG response was determined by ELISA. Briefly, 96 well ELISA plate was covered with 100 ng/well of OVA protein diluted in 100 µl of PBS and 3 of wells were used for negative control which was included only PBS. The plate was sealed and incubated at 4°C for ON. Each well was aspirated and washed with 300 µl of wash buffer (PBS-T) for 3 times. After the last wash, remaining wash buffers and bubbles were removed by blotting the plate against clean towel papers. 200 µl of reagent diluent (1% BSA in PBS) was added to each well and the plate was incubated at RT for minimum 1 hour, maximum 2 hours. The washing step was repeated. After the last wash, remaining wash buffers and bubbles were removed by blotting the plate against clean towel papers. 100 µl of standards (from 25 pg/ml to 5000 pg/ml), or samples (1:500 dilution) were added and the plate was covered with stretch film and incubated at RT for 1 hour. The washing step was repeated and after the last wash, the same cleaning protocol was performed. 100 µl of 1:2000 anti-mouse IgG HRP linked secondary antibody was added to each well and the plate was covered with stretch film and incubate at RT for 1 hour. The washing step was repeated and after the last wash, the same cleaning protocol was performed. 100 µl of substrate mixture (1:1 mixture of color reagent A and B) was added to each well avoiding the plate from the direct light and the plate was incubated at RT for 20 minutes. Then, 20 µl of stop solution (2N H₂SO₄) was added to each well. The plate was gently tapped to ensure thorough mixing. The absorbance of each well was measured at 450 and 540 nm via plate reader.

### 4. Tracking of mCherry-ASC Specks in vivo

mCherry-ASC specks were used to track ASC specks after intraperitoneal injections. 8 C57BL/6 mice were intraperitoneally injected with 10 µg of mCherry-ASC in 300 µl PBS. As a control 10 µg mCherry in 300 µl of PBS and only 300 µl of PBS were injected as control groups. 8 and 4 mice were used mCherry and PBS injections, respectively.

### 4.1. In vivo Imaging

IVIS Spectrum in vivo imaging system was used to visualize injected mCherry-ASC specks in vivo and at day 1, 2 and 10, animals were monitored under IVIS Spectrum machine.

### 4.2. Tissue Sampling for Western blot and Immunohistochemistry

At day 2, half of the animals from each group was sacrificed and at day 10 the other half was sacrificed. Spleen, mesenteric lymph nodes, liver and brain of the animals were excised. Spleens were monitored ex vivo under IVIS. Representative parts from spleen were picked for Western blot analysis and the other parts were embedded in OCT compound and stored at - 80°C. Mesenteric lymph nodes, liver and brain of the animals were kept at -80°C for further Western blot analysis.

### 5. Western Blot Analysis

### 5.1. Sample Preparation from Cell Culture

Expression pattern and regulation of genes in protein level is generally detected by Western blot analysis. To analyze expression of proteins in cell, each well of 6 well plate was washed with 1 ml PBS or cells were centrifuged and washed with 1ml PBS for adherent and suspension cell cultures, respectively. After aspiration of PBS, 160 µl of proatease inhibitor complex supplemented 0.2% NP40 lysis buffer was added onto cells. For adherent cell lines, cells were collected with scraper and micropipette in a 1.5 ml tube. Collected cells were kept on ice for 1 hour and vortexed for each 15 minutes for complete lyse of the cells. Larger fragments of the cells were pelleted by centrifugation at 13000 rpm at 4°C for 30 min. Supernatant was taken to another 1.5 ml tube. Then, 40 µl of 5X Laemmli buffer was added on the sample and proteins were denatured at 95°C for 10 min in Laemmli buffer.

### 5.2. Sample Preparation from Tissue

200 µl of lysis buffer added on minced and frozen representative parts of large tissues (spleen, liver, brain and tumor) and whole mesenteric lymph node in 2 ml tube. Tissues were lysed via sonication for 50% of 1 min under 70% power and kept on ice for 15 min. Then tissue lysate was centrifuged at 13000 rpm for 30 min to remove larger fragments and connective tissue parts. Supernatant was taken into a new 1.5 ml tube and diluted. Then, 5X Laemmli buffer was added appropriately and proteins were denatured at 95°C for 15 min.

### 5.3. SDS-PAGE Gel Preparation

8 ml of 10%, %12 or 15% resolving gel solution was mixed with 80 µl of 10% APS and 8 µl of TEMED. After mixing the solution, gel was loaded between 1.5 mm spacer and short plate. Then, isopropanol was covered onto gel for at surface. After polymerization of resolving gel, isopropanol removed and 4 ml of 4% stacking gel was prepared by mixing 40 µl of 10% APS and 4 µl of TEMED. Stacking gel was loaded on resolving gel and 10 well comb was inserted. When polymerization of stacking gel was completed, SDS-PAGE gel was used for protein gel electrophoresis.

### 5.4. Protein Gel Electrophoresis

Proteins was loaded to SDS-PAGE gel to separate them according to their kDa. Casted SDS-PAGE gel was placed in vertical protein gel electrophoresis tank and tank was filled with running buffer. Comb was carefully was taken out and 35 µl of denatured protein samples in Leamli buffer and protein ladder were loaded into wells. Samples were run at 70V until they pass stacking gel and run at 120 V till dye comes to bottom of the resolving gel.

### 5.5. Semi-dry Transfer

Proteins on SDS-PAGE gel was transferred to nitrocellulose membrane for antibody blotting. Briefly, nitrocellulose membrane was activated in methanol and the membrane and 2 filter paper were wet in transfer buffer. 1 filter paper, the membrane, SDS-PAGE gel and the other filter paper were placed onto semi-dry transfer apparatus, respectively. Cover of the apparatus was closed and transfer of proteins from SDS-PAGE to nitrocellulose membrane was completed at 10V for 50 min.

### 5.6. Membrane Blocking

Protein transferred membranes was taken into a plastic container and blocked with 5% (w/v) skim milk powder in TBS-T in for 1 hour to prevent non-specific binding of detection antibodies.

### 5.7. Antibody Incubations

Primary antibodies were diluted in 5% (w/v) BSA in TBS-T and sodium azide was added to antibody solutions to inhibit microbial growth at 4°C. After blocking of membranes, they were washed 3 times with TBS-T for 5 min. The membranes were incubated with primary antibodies at appropriate dilutions at 4°C overnight. Prepared antibody dilutions were kept at 4°C and used 5-10 times for incubations. Primary antibody incubated membranes were washed 3 times with TBS-T for 5 min. Secondary antibody solutions were prepared by diluting HRP conjugated anti-mouse or anti-rabbit antibodies 1/2000 (v/v) in 5% (w/v) skim milk powder in TBS-T. The membranes were incubated with secondary antibody dilutions for 1 hour at RT.

### 5.8. Visualization of Proteins

The membranes were washed 3 times with TBS-T for 5 min after secondary antibody incubation and they were oat in 2 ml of 1:1 mixed ECL Western Blotting substrates. Then, protein bands on the membranes were visualized using Syngene Gbox Chemi chemiluminescence imaging device.

### 6. Cell Surface Staining and Flow Cytometry Analysis

1 million cells were washed with 3 ml of FACS buffer twice in FACS tube. Supernatant was discarded and 5 µl of Fc blocker and 10 µl of flourochrome conjugated antibodies were added. Cells were incubated with antibodies for 30 minutes at +4°C. Cells were again washed three times with 3 ml FACS buffer. Supernatant was discarded and cells were re-suspended in 1 ml of FACS buffer. Upregulation of cell surface markers was analyzed with BD Acuri and FlowJo software.

## Claims

1. A composition comprising a tumor antigen and an ASC speck formed by Apoptosis Associated speck-like Protein containing CARD (ASC protein) for use in a method of cancer therapy.

2. A composition for use according to claim 1 wherein said method comprises inducing and/or stimulating an immune response against a cancer disease in a mammal, wherein said induction or stimulation causes a decrease in cancer progression or growth arrest of cancer cells.

3. A composition for use according to claims 1 or 2 wherein the cancer is thymus cancer.

4. A composition for use according to any one of claims 1 to 3 wherein the tumor antigen is carried by the ASC speck by forming hydrophobic interactions with the ASC proteins forming the ASC speck.

5. A composition for use according to any one of claims 1 to 3 wherein the tumor antigen is carried by the ASC speck as being a part of a fusion protein formed by said tumor antigen and at least one ASC protein forming the ASC speck.

6. A composition for use according to claim 5 wherein the tumor antigen being fused to the ASC protein at the N-terminus or C-terminus.

7. A composition for use according to any one of claims 1 to 6 wherein the tumor antigen being carried inside the ASC speck.

8. A composition for use according to any one of claims 1 to 7 wherein the cancer is a tumor forming cancer and the method comprises a decrease in tumor progression or a decrease in tumor size.

9. A composition for generating an immune response to cancer in a mammal, comprising a tumor antigen and an ASC speck formed by ASC proteins.

10. A composition according to claim 9 wherein the tumor antigen is carried by the ASC speck as being a part of a fusion protein formed by said tumor antigen and at least one ASC protein forming the ASC speck.

## Patentansprüche

1. Eine Zusammensetzung, die ein Tumorantigen und einen ASC-Speck umfasst, der aus einem Apoptose-assoziierten, speckartigen Protein mit einer CARD-Domäne (ASC-Protein) gebildet wird, zur Verwendung in einem Verfahren zur Krebstherapie.

2. Eine Zusammensetzung zur Verwendung nach Anspruch 1, wobei das Verfahren das Induzieren und/oder Stimulieren einer Immunantwort gegen eine Krebserkrankung bei einem Säugetier umfasst, wobei die besagte Induktion oder Stimulation eine Verringerung der Krebsprogression oder Wachstumsstillstand von Krebszellen bewirkt.

3. Eine Zusammensetzung zur Verwendung nach den Ansprüchen 1 oder 2, wobei der Krebs Thymuskrebs ist.

4. Eine Zusammensetzung zur Verwendung nach irgendeinem der Ansprüche 1 bis 3, wobei das Tumorantigen von dem ASC-Speck getragen wird durch Bilden von hydrophoben Wechselwirkungen mit den ASC-Proteinen, die den ASC-Speck bilden.

5. Eine Zusammensetzung zur Verwendung nach irgendeinem der Ansprüche 1 bis 3, wobei das Tumorantigen von dem ASC-Speck als Teil eines Fusionsproteins getragen wird, das von dem besagten Tumorantigen und mindestens einem den ASC-Speck bildenden ASC-Protein gebildet wird.

6. Eine Zusammensetzung zur Verwendung nach Anspruch 5, wobei das Tumorantigen am N-Terminus oder C-Terminus mit dem ASC-Protein fusioniert ist.

7. Eine Zusammensetzung zur Verwendung nach irgendeinem der Ansprüche 1 bis 6, wobei das Tumorantigen innerhalb des ASC-Specks getragen wird.

8. Eine Zusammensetzung zur Verwendung nach irgendeinem der Ansprüche 1 bis 7, wobei der Krebs ein tumorbildender Krebs ist und das Verfahren eine Verringerung der Tumorprogression oder eine Verringerung der Tumorgröße umfasst.

9. Eine Zusammensetzung zum Erzeugen einer Immunantwort auf Krebs bei einem Säugetier, die ein Tumorantigen und einen von ASC-Proteinen gebildeten ASC-Speck umfasst.

10. Eine Zusammensetzung nach Anspruch 9, wobei das Tumorantigen von dem ASC-Speck als Teil eines Fusionsproteins getragen wird, das von dem Tumorantigen und mindestens einem den ASC-Speck bildenden ASC-Protein gebildet wird.

## Revendications

1. Composition comprenant un antigène tumoral et une tache ASC formée par une Protéine de type tache Associée à l'Apoptose contenant un domaine CARD (protéine ASC) pour une utilisation dans un procédé de traitement du cancer.

2. Composition pour une utilisation selon la revendication 1 dans laquelle ledit procédé comprend l'induction et/ou la stimulation d'une réponse immunitaire contre une maladie cancéreuse chez un mammifère, dans laquelle ladite induction ou stimulation provoque une diminution de la progression du cancer ou un arrêt de la croissance des cellules cancéreuses.

3. Composition pour une utilisation selon les revendications 1 ou 2 dans laquelle le cancer est un cancer du thymus.

4. Composition pour une utilisation selon l'une quelconque des revendications 1 à 3 dans laquelle l'antigène tumoral est porté par la tache ASC en formant des interactions hydrophobes avec les protéines ASC formant la tache ASC.

5. Composition pour une utilisation selon l'une quelconque des revendications 1 à 3 dans laquelle l'antigène tumoral est porté par la tache ASC en tant que partie d'une protéine de fusion formée par ledit antigène tumoral et au moins une protéine ASC formant la tache ASC.

6. Composition pour une utilisation selon la revendication 5 dans laquelle l'antigène tumoral est fusionné à la protéine ASC au niveau de l'extrémité N ou de l'extrémité C.

7. Composition pour une utilisation selon l'une quelconque des revendications 1 à 6 dans laquelle l'antigène tumoral est porté à l'intérieur de la tache ASC.

8. Composition pour une utilisation selon l'une quelconque des revendications 1 à 7 dans laquelle le cancer est un cancer formant une tumeur et le procédé comprend une diminution de la progression tumorale ou une diminution de la taille tumorale.

9. Composition pour générer une réponse immunitaire au cancer chez un mammifère, comprenant un antigène tumoral et une tache ASC formée par des protéines ASC.

10. Composition selon la revendication 9 dans laquelle l'antigène tumoral est porté par la tache ASC en tant que partie d'une protéine de fusion formée par ledit antigène tumoral et au moins une protéine ASC formant la tache ASC.
